# EUROPEAN PATENT SPECIFICATION

(11) **EP 4 337 673 B1**
(45) Date of publication and mention of the grant of the patent: **09.07.2025**
(21) Application number: 22728608.5
(22) Date of filing: 12.05.2022
(51) Int. Cl.: C07K 14/47, A61K 47/64, A61K 38/00, A61P 9/10

(54) **PSD-95 INHIBITORS AND USES THEREOF**
PSD-95-INHIBITOREN UND VERWENDUNGEN DAVON
INHIBITEURS DE PSD-95 ET UTILISATIONS ASSOCIÉES

(30) Priority: 12.05.2021 EP 21173587
(43) Date of publication of application: 20.03.2024
(73) Proprietor: University of Copenhagen, 1165 Copenhagen K (DK)
(72) Inventor: SEREIKAITE, Vita, 2000 Frederiksberg (DK); BECH-BARTLING, Christian Reinhard Otto, 2100 Copenhagen Ø (DK); STRØMGAARD, Kristian, 4000 Roskilde (DK)
(74) Representative: Høiberg P/S
(86) International application number: PCT/EP2022/062893
(87) International publication number: WO 2022/238530

(56) References cited:
- WO-A1-2012/156308
- WO-A1-2020/083905
- WENDER P A ET AL: "The design, synthesis, and evaluation of molecules that enable or enhance cellular uptake: Peptoid molecular transporters", PROCEEDINGS OF THE NATIONAL ACADEMY OF SCIENCES, NATIONAL ACADEMY OF SCIENCES, US, vol. 97, no. 24, 21 November 2000 (2000-11-21), pages 13003 - 13008, XP002247290, ISSN: 0027-8424, DOI: 10.1073/PNAS.97.24.13003
- FUTAKI SHIROH ET AL: "Arginine-rich peptides: An abundant source of membrane-permeable peptides having potential as carriers for intracellular protein delivery", JOURNAL OF BIOLOGICAL CHEMISTRY, AMERICAN SOCIETY FOR BIOCHEMISTRY AND MOLECULAR BIOLOGY, US, vol. 276, no. 8, 17 November 2000 (2000-11-17), pages 5836 - 5840, XP002210171, ISSN: 0021-9258, DOI: 10.1074/JBC.M007540200

## Description

### Field of invention

The present invention relates to compounds capable of binding to the PDZ domains of PSD-95 and their medical use as inhibitors of protein-protein interaction mediated by PSD-95.

### Background

PSD-95 is a scaffolding protein in neuronal synapses that interacts with N-methyl-D-aspartate (NMDA) receptors (Kornau et al. 1995) and neuronal nitric oxide synthase (nNOS) through its PSD-95/Discs-large/ZO-1 (PDZ) domains. During cerebral ischemia, excessive glutamate release leads to hyperactivation of the NMDA receptors and harmful elevation of intracellular levels of Ca²⁺ and NO, which ultimately induces excitotoxicity, leading to neuronal death and brain damage (Aarts et al., 2002; Dawson et al., 1991; Huang et al., 1994; Sattler et al., 1999). PSD-95 is being explored as a drug target in acute ischemic stroke (AIS) and related ischemic conditions in the brain.

The 20-mer peptide nerinetide (also known as Tat-NR2B9c or NA-1) was recently examined in a Phase 3 clinical trial ESCAPE-NA1 (Hill et al., 2020), which evaluated whether treatment with nerinetide, in addition to standard of care such as alteplase (tissue plasminogen activator, tPA), would improve clinical outcomes for patients with ischemic stroke and who were undergoing endovascular thrombectomy (Hill et al., 2020). A total of 1105 patients were included in the study, where 659 patients received tPA treatment, and 446 patients did not. It was found that, while nerinetide was not effective in patients receiving alteplase, in the group not receiving alteplase, nerinetide resulted in improved functional outcomes, reduced mortality, and reduced infarction volumes (Hill et al., 2020). It was recently demonstrated that the lack of effect in the tPA-treated group could be explained by a drug-drug interaction between nerinetide and tPA. Specifically, tPA generates plasmin, a serine protease which cleaves nerinetide (Mayor-Nunez et al. 2021). Thus, it is highly advantageous that a drug is compatible with administration of thrombolytic agents, which is standard of care for AIS.

Nerinetide suffers from a relatively low affinity to the target PSD-95, which has prompted the design of dimeric compounds such as Tat-N-dimer and O-dimer, also known as UCCB01-144 (AB144) and UCCB01-125, respectively (Bach et al., 2009, 2012; WO 2010/004003, WO 2012/156308; Kucharz et al., 2017). In an in vitro fluorescence polarization (FP) assay the *Kᵢ* values of nerinetide towards PDZ1 and PDZ2 of PSD-95 were 5-10 µM (Bach et al., 2012). In contrast, AB144 and UCCB01-125, due to their dimeric structures and bivalent properties, bind PDZ1 and PDZ2 of PSD-95 simultaneously, leading to a 500-1000-fold higher affinity towards PDZ1-2 (*Kᵢ* values of 4.6 and 9.5 nM, respectively) relative to that of monomeric nerinetide (Bach et al., 2012). In mice that underwent permanent middle cerebral artery occlusion (pMCAO), a single intravenous (i.v.) bolus injection of AB144 (3 nmol/g) given 30 min post-ischemia reduced the infarct volumes by 40% and 37% at 6 h and 48 h of the post-ischemic survival period, respectively, compared to the effect of saline (Bach et al., 2012). Furthermore, functional outcomes such as grip strength and rotarod performance were improved and thus correlated with reductions in infarct size. Under the same experimental conditions and dose (3 nmol/g), nerinetide did not show significant neuroprotective properties (Bach et al., 2012). Likewise, UCCB01-125 without the Tat moiety did not reach the brain and showed no neuroprotective properties (Bach et al., 2012).

PSD-95 is therefore considered a particular promising drug target for the treatment of acute conditions such as subarachnoid hemorrhage (SAH) and AIS. Importantly, PSD-95 is located intracellularly, thus any drug targeting PSD-95 needs to efficiently cross the cell membrane and bind to PSD-95.

### Summary

The present inventors have developed a series of novel compounds with high affinity for PSD-95. As compared to known PSD-95 inhibitors like nerinetide, the compounds of the present invention have improved cellular uptake and much higher plasmin stability. Thus, the compounds of the present invention are useful in the treatment of excitotoxic-related diseases such as AIS and SAH, and are compatible with standard of care, such as thrombolytic agents.

In one aspect, the present invention relates to a PSD-95 inhibitor comprising:
a. a first peptide (P₁) comprising or consisting of the amino acid sequence
   X₂TX₃V (SEQ ID NO: 53), wherein
   X₂ is selected from the group consisting of E and S;
   X₃ is selected from the group consisting of L, T, V, R and D;
b. a second peptide (P₂) comprising or consisting of the amino acid sequence X₅TX₆V (SEQ ID NO: 55), wherein
   X₅ is selected from the group consisting of E and S;
   X₆ is selected from the group consisting of L, T, V, R and D; and
c. a Cell Penetrating Peptide (CPP) selected from the group consisting of:
   i. a poly-L-arginine peptide (poly-Arg) consisting of 3 to 9 L-arginine residues;
   ii. yGrkkrrqrrr (SEQ ID NO: 9, D-TAT);
   iii. RQIKIWFQNRRMKWKK (SEQ ID NO: 14, L-Pen);
   iv. rqikiwfqnrrmkwkk (SEQ ID NO: 15, D-Pen);
   v. RKKRRRESRKKRRRES (SEQ ID NO: 17, L-DPV3);
   vi. LLIILRRRIRKQAHAHSK (SEQ ID NO: 18, L-pVEC);
   vii. KLALKLALKALKAALKLA (SEQ ID NO: 16, L-MAP);
   viii. PLIYLRLLRGQF (SEQ ID NO: 19, L-TP2);
   ix. |(Dap)KAPETALD| (SEQ ID NO: 21, MiniAp4); and
   x. |Ff(Nal2)RrRrQ|GABA-K (SEQ ID NO: 22, CPP12),
wherein the CPP is linked to a linker via its C-terminal, and P₁ and P₂ are conjugated to the linker via their N-termini, and the compound has the general structure of Formula (I): 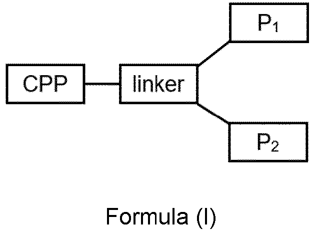 or a pharmaceutically acceptable salt thereof.

In another aspect, the present invention relates to a compound of the invention for use as a medicament.

In another aspect, the present invention relates to a compound of the invention for use in treating, preventing, reducing and/or delaying development of an excitotoxic-related disease such as stroke, for example selected from acute ischemic stroke and subarachnoid hemorrhage.

Furthermore, the present invention relates to a method for manufacturing a compound of the invention.

The invention is defined by the claims. Any subject matter falling outside the scope of the claims is provided for information purposes only.

### Description of Drawings

**Figure 1****.** Affinity of *O*-PEG₄ dimers (compounds 1 to 7) towards PSD-95 PDZ1-2 as determined by FP. Data presented as mean + SEM, n = 3.
**Figure 2****.** Affinity of dimeric ligands fused to Ac-TAT and Ac-polyArg towards PSD-95 PDZ1-2 as determined by FP. Data presented as mean + SEM, n = 3.
**Figure 3****.** Affinity of *N*-PEG₄(IETDV)₂ fused to various CPP-tags (SEQ IDs 8 to 22) towards PSD-95 PDZ1-2 as determined by FP. Data presented as mean + SEM, n = 3.
**Figure 4****.** Affinity of *N*PEG₄-(KETLV)₂ dimeric ligands fused to polyArg CPP-tags towards PSD-95 PDZ1-2 as determined by FP. Data presented as mean + SEM, n = 3.
**Figure 5****.** Half-life of dimeric ligands fused to Ac-TAT and Ac-polyArg as determined in *in vitro* plasmin stability assay. Data presented as mean + SEM, n = 3.
**Figure 6****.** Half-life of *N*-PEG₄(IETDV)₂ fused to various CPP-tags (SEQ IDs 8 to 22) as determined in *in vitro* plasmin stability assay. Data presented as mean + SEM, n = 3.
**Figure 7****.** Cellular uptake of dimeric ligands fused to Ac-TAT and Ac-polyArg as determined by CAPA. Data presented as mean + SEM, n = 3.
**Figure 8****.** Cellular uptake of *N*-PEG₄(IETDV)₂ fused to various CPP-tags (SEQ IDs 8 to 22) as determined by CAPA. Data presented as mean + SEM, n = 3.
**Figure 9****.** Cellular uptake of *N*-PEG₄(IETDV)₂ and *N*-PEG₄(KETLV)₂ fused to various polyArg CPP-tags as determined by CAPA. Data presented as mean + SEM, n = 3.
**Figure 10****.** Half-life of *N*-PEG₄(IETDV)₂ and *N*-PEG₄(KETLV)₂ fused to polyArg CPP-tags as determined in human plasma stability assay. Data presented as mean ± SEM, n = 3.
**Figure 11****.** Generic structure of compounds binding to PDZ1-2 of PSD-95. R₁ through R₅ are amino acid side-chains as described herein and R₆ represents a CPP-tag as described herein.

### Detailed description

### Definitions

Amide bond: The term 'amide bond' as used herein is a chemical bond formed by a reaction between a carboxylic acid and an amine (and concomitant elimination of water). Where the reaction is between two amino acid residues, the bond formed as a result of the reaction is known as a peptide linkage (peptide bond).

Comprising: The term 'comprising' as used herein should be understood in an inclusive manner. Hence, by way of example, a composition comprising compound X, may comprise compound X and optionally additional compounds.

Dimer: The term dimer as used herein refers to two identical or non-identical chemical moieties associated by chemical or physical interaction. By way of example, the dimer can be a homodimer such as two identical chemical moieties linked by a linker. The dimer may also be a heterodimer such as two different chemical moieties linked by a linker. An example of a dimer is a PSD-95 inhibitor of the present invention which is a compound comprising two peptides that are covalently linked by means of a linker, wherein the peptides are capable of binding to, or interacting with, PDZ1 and PDZ2 domains of PSD-95 simultaneously.

Dipeptide: The term 'dipeptide' as used herein refers to two natural or non-natural amino acids linked by a peptide bond.

Fatty acid: The term fatty acid (abbreviated FA) as used herein typically refers to a carboxylic acid with a long aliphatic carbon chain, which can be either saturated or unsaturated. The fatty acid can be selected from Short-chain fatty acids (SCFA), Medium-chain fatty acids (MCFA), Long-chain fatty acids (LCFA) and Very long chain fatty acids (VLCFA). Short-chain fatty acids (SCFA) are fatty acids with aliphatic tails of fewer than six carbons (i.e. butyric acid). Medium-chain fatty acids (MCFA) are fatty acids with aliphatic tails of 6-12 carbons, which can form medium-chain triglycerides. Long-chain fatty acids (LCFA) are fatty acids with aliphatic tails 13 to 21 carbons. Very long chain fatty acids (VLCFA) are fatty acids with aliphatic tails longer than 22 carbons. The fatty acid of the present invention can be any suitable fatty acid or fatty acid derivative known by those of skill in the art.

Non-proteinogenic amino acids: Non-proteinogenic amino acids also referred to as non-coded, non-standard or non-natural amino acids are amino acids which are not encoded by the genetic code. A non-exhaustive list of non-proteinogenic amino acids include γ-aminobutyric acid, L-3-(2-naphthyl)alanine, L-2,3-diaminopropionic acid, α-amino-n-butyric acid, norvaline, norleucine, isoleucine, alloisoleucine, tert-leucine, α-amino-n-heptanoic acid, pipecolic acid, α,β-diaminopropionic acid, α,γ-diaminobutyric acid, ornithine, allothreonine, homocysteine, homoserine, β-alanine, β-amino-n-butyric acid, β-aminoisobutyric acid, α-aminoisobutyric acid, isovaline, sarcosine, N-ethyl glycine, N-propyl glycine, N-isopropyl glycine, N-methyl alanine, N-ethyl alanine, N-methyl β-alanine, N-ethyl β-alanine, isoserine and α-hydroxy-γ-aminobutyric acid.

Proteinogenic amino acids: Proteinogenic amino acids, also referred to as natural amino acids, include alanine, cysteine, selenocysteine, aspartic acid, glutamic acid, phenylalanine, glycine, histidine, isoleucine, lysine, leucine, methionine, asparagine, proline, pyrrolysine, glutamine, arginine, serine, threonine, valine, tryptophan and tyrosine. Capital letter abbreviations indicate L-amino acids, whereas lower case letter abbreviations indicate D-amino acids.

PDZ: The term 'PDZ' as used herein refers to Postsynaptic density protein-95 (PSD-95), Drosophila homologue discs large tumor suppressor (DIgA), Zonula occludens-1 protein (zo-1).

PSD-95: The term 'PSD-95' as used herein refers to postsynaptic density protein-95.

PSD-95 inhibitor: The term 'PSD-95 inhibitor' as used herein refers to a compound that binds to PDZ1, PDZ2, or both PDZ1 and PDZ2 of PSD-95 and inhibits the protein-protein interactions facilitated by these PDZ domains in a cell. An example of an interaction that is inhibited by a PSD-95 inhibitor is the ternary complex formation between nNOS, PSD-95 and the NMDA receptor.

### Compounds

In one aspect, the present invention concerns a PSD-95 inhibitor comprising a first peptide (P₁) linked to a Cell Penetrating Peptide (CPP). P₁ is linked to the CPP via a linker. The compound further comprises a second peptide (P₂). The CPP is linked to P₁ and P₂ via the linker. Thus, the CPP is linked to the linker via its C-terminal, and P₁ and P₂ are conjugated to a linker via their N-termini, and the compound has the following general structure of Formula (I):

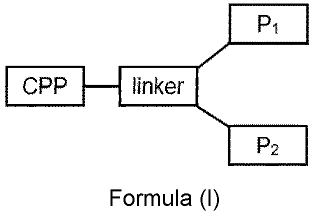

Thus, in one embodiment, the compound of the present invention comprises a first peptide (P₁) and a second peptide (P₂), wherein both P₁ and P₂ are able to simultaneously bind to the PDZ1 and PDZ2 domains of PSD-95, i.e. the compound is a dimeric PSD-95 inhibitor.

P₁ is a peptide comprising or consisting of the amino acid sequence X₂TX₃V (SEQ ID NO: 53), wherein
a. X₂ is selected from the group consisting of E and S; and
b. X₃ is selected from the group consisting of L, T, V, R and D,
and wherein P₁ is linked to a Cell Penetrating Peptide (CPP).

The compound of the present invention comprises:
a. a first peptide (P₁) comprising or consisting of the amino acid sequence X₂TX₃V (SEQ ID NO: 53), wherein
   X₂ is selected from the group consisting of E and S;
   X₃ is selected from the group consisting of L, T, V, R and D;
b. a second peptide (P₂) comprising or consisting of the amino acid sequence X₅TX₆V (SEQ ID NO: 55), wherein
   X₅ is selected from the group consisting of E and S;
   X₆ is selected from the group consisting of L, T, V, R and D; and
c. a Cell Penetrating Peptide (CPP) selected from the group consisting of:
   i. a poly-L-arginine peptide (poly-Arg) consisting of 3 to 9 L-arginine residues;
   ii. yGrkkrrqrrr (SEQ ID NO: 9, D-TAT);
   iii. RQIKIWFQNRRMKWKK (SEQ ID NO: 14, L-Pen);
   iv. rqikiwfqnrrmkwkk (SEQ ID NO: 15, D-Pen);
   v. RKKRRRESRKKRRRES (SEQ ID NO: 17, L-DPV3);
   vi. LLIILRRRIRKQAHAHSK (SEQ ID NO: 18, L-pVEC);
   vii. KLALKLALKALKAALKLA (SEQ ID NO: 16, L-MAP);
   viii. PLIYLRLLRGQF (SEQ ID NO: 19, L-TP2);
   ix. |(Dap)KAPETALD| (SEQ ID NO: 21, MiniAp4); and
   x. |Ff(Nal2)RrRrQ|GABA-K (SEQ ID NO: 22, CPP12),
wherein the CPP is linked to a linker via its C-terminal, and P₁ and P₂ are conjugated to the linker via their N-termini, and the compound has the general structure of Formula (I): 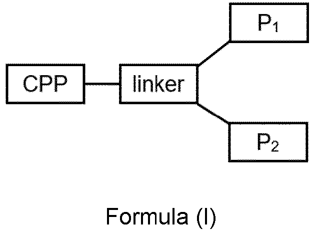 or a pharmaceutically acceptable salt thereof.

### Binding peptides P₁ and P₂

The peptides P₁ and P₂ have high affinity for PDZ1 and PDZ2 domains of PSD-95. The compound of the present invention is a PSD-95 inhibitor, i.e. the compound is capable of binding to one or more of the PDZ domains of PSD-95.

The compound comprises a first peptide (P₁) comprising or consisting of the amino acid sequence X₂TX₃V (SEQ ID NO: 53), wherein
a. X₂ is selected from the group consisting of E and S; and
b. X₃ is selected from the group consisting of L, T, V, R and D.

In one embodiment, P₁ comprises or consists of the sequence X₁X₂TX₃V (SEQ ID NO: 54), wherein
a. X₁ is selected from the group consisting of K, V and I;
b. X₂ is selected from the group consisting of E and S; and
c. X₃ is selected from the group consisting of L, T, V, R and D.

In one embodiment, P₁ comprises or consists of IETDV (SEQ ID NO: 1). In one embodiment, P₁ comprises or consists of KETLV (SEQ ID NO: 2). In one embodiment, P₁ comprises or consists of KETTV (SEQ ID NO: 3). In one embodiment, P₁ comprises or consists of KETVV (SEQ ID NO: 4). In one embodiment, P₁ comprises or consists of KETRV (SEQ ID NO: 5). In one embodiment, P₁ comprises or consists of ISTDV (SEQ ID NO: 6). In one embodiment, P₁ comprises or consists of VETVV (SEQ ID NO: 7).

In one embodiment, P₁ consists of 4 to 10 amino acid residues, such as 5 amino acid residues, such as 6 amino acid residues. The amino acid residues in excess of the amino acid residues given in SEQ ID NO: 53 or SEQ ID NO: 54 may be proteinogenic or non-proteinogenic amino acids.

The compound further comprises a second peptide (P₂) comprising or consisting of the amino acid sequence X₅TX₆V (SEQ ID NO: 55), wherein
a. X₅ is selected from the group consisting of E and S; and
b. X₆ is selected from the group consisting of L, T, V, R and D.

In one embodiment, P₂ comprises or consists of the sequence X₄X₅TX₆V (SEQ ID NO: 56), wherein
a. X₄ is selected from the group consisting of K, V and I;
b. X₅ is selected from the group consisting of E and S; and
c. X₆ is selected from the group consisting of L, T, V, R and D.

In one embodiment, P₂ comprises or consists of IETDV (SEQ ID NO: 1). In one embodiment, P₂ comprises or consists of KETLV (SEQ ID NO: 2). In one embodiment, P₂ comprises or consists of KETTV (SEQ ID NO: 3). In one embodiment, P₂ comprises or consists of KETVV (SEQ ID NO: 4). In one embodiment, P₂ comprises or consists of KETRV (SEQ ID NO: 5). In one embodiment, P₂ comprises or consists of ISTDV (SEQ ID NO: 6). In one embodiment, P₂ comprises or consists of VETVV (SEQ ID NO: 7).

In one embodiment, P₂ consists of 4 to 10 amino acid residues, such as 5 amino acid residues, such as 6 amino acid residues. The amino acid residues in excess of the amino acid residues given in SEQ ID NO: 55 or SEQ ID NO: 56 may be proteinogenic or non-proteinogenic amino acids.

In one embodiment, P₁ and P₂ are identical.

In one embodiment, when X₂ is E, then X₃ is not D. In one embodiment, when X₅ is E, then X₆ is not D. In one embodiment, P₁ and/or P₂ are not IETDV (SEQ ID NO: 1).

The peptides P₁ and P₂ are conjugated via their N-termini to a linker or a CPP. In one embodiment, the amino acid sequence X₂TX₃V (SEQ ID NO: 53) is the C-terminal of P₁. In one embodiment, the amino acid sequence X₅TX₆V (SEQ ID NO: 55) is the C-terminal of P₂.

### Cell Penetrating Peptide (CPP)

The compounds of the present invention comprises peptides P₁ and P₂ linked to a Cell Penetrating Peptide (CPP). A CPP is characterised by the ability to cross the blood brain barrier (BBB) and/or the plasma membrane of mammalian cells, and thereby may give rise to the intracellular delivery of cargo molecules, such as peptides, proteins, oligonucleotides to which it is linked.

In one embodiment, the CPP is a poly-L-arginine peptide (poly-Arg) consisting of 3 to 9 L-arginine residues. In one embodiment, the poly-Arg consists of 8 or 9 L-arginine residues. In one embodiment, the CPP comprises or consists of the amino acid sequence RRRRRRRRR (L-Arg₉, SEQ ID NO: 12). In one embodiment, the CPP comprises or consists of the amino acid sequence RRRRRRRR (L-Arg₈, SEQ ID NO: 57). In one embodiment, the CPP comprises or consists of the amino acid sequence RRRRRRR (L-Arg₇, SEQ ID NO: 58). In one embodiment, the CPP comprises or consists of the amino acid sequence RRRRRR (L-Arg₆, SEQ ID NO: 59). In one embodiment, the CPP comprises or consists of the amino acid sequence RRRRR (L-Arg₅, SEQ ID NO: 60). In one embodiment, the CPP comprises or consists of the amino acid sequence RRRR (L-Arg₄, SEQ ID NO: 61). In one embodiment, the CPP comprises or consists of the amino acid sequence RRR (L-Arg₃).

The CPP is selected from the CPPs L-Arg₉, L-Arg₈, L-Arg₇, L-Arg₆, L-Arg₅, L-Arg₄, L-Arg₃, D-TAT, L-Pen, D-Pen, L-DPV3, L-pVEC, L-MAP, L-TP2, MiniAp4, and CPP12 given in table 1.

**Table 1. Cell Penetrating Peptides.**

| **Name** | **Sequence** | **SEQ ID NO:** |
|---|---|---|
| L-TAT | YGRKKRRQRRR | 8 |
| D-TAT | yGrkkrrqrrr | 9 |
| mTAT | rRrGrKkRr | 10 |
| riTAT | rrrqrrkkr | 11 |
| L-Arg₉ | RRRRRRRRR | 12 |
| L-Arg₈ | RRRRRRRR | 57 |
| L-Arg₇ | RRRRRRR | 58 |
| L-Arg₆ | RRRRRR | 59 |
| L-Arg₅ | RRRRR | 60 |
| L-Arg₄ | RRRR | 61 |
| L-Arg₃ | RRR | |
| D-Arg₉ | rrrrrrrrr | 13 |
| L-Pen | RQIKIWFQNRRMKWKK | 14 |
| D-Pen | rqikiwfqnrrmkwkk | 15 |
| L-MAP | KLALKLALKALKAALKLA | 16 |
| L-DPV3 | RKKRRRESRKKRRRES | 17 |
| L-pVEC | LLIILRRRIRKQAHAHSK | 18 |
| L-TP2 | PLIYLRLLRGQF | 19 |
| D-TP2 | pliylrllrGqf | 20 |
| MiniAp4 | \|(Dap)KAPETALD\| | 21 |
| CPP12 | \|Ff(Nal2)RrRrQ\|GABA-K | 22 |

In one embodiment, the CPP comprises a peptide selected from the group consisting of poly-L-arginine peptide (poly-Arg) consisting of 3 to 9 L-arginine residues, such as L-Arg₉; D-TAT; L-Pen; D-Pen; L-DPV3; L-pVEC; L-MAP; L-TP2; MiniAp4 and CPP12. In one embodiment, the CPP is selected from the group consisting of poly-L-arginine peptide (poly-Arg) consisting of 3 to 9 L-arginine residues, such as L-Arg₉; D-TAT; L-Pen; D-Pen; L-DPV3; L-pVEC; L-MAP; L-TP2; MiniAp4 and CPP12. In one embodiment, the CPP is D-TAT. In one embodiment, the CPP is L-Pen. In one embodiment, the CPP is D-Pen. In one embodiment, the CPP is L-DPV3. In one embodiment, the CPP is L-pVEC. In one embodiment, the CPP is L-MAP. In one embodiment, the CPP is L-TP2. In one embodiment, the CPP is MiniAp4. In one embodiment, the CPP is CPP12.

In one embodiment, the CPP comprises no more than 20 amino acid residues, such as no more than 19, such as no more than 18, such as no more than 17, such as no more than 16, such as no more than 15, such as no more than 14, such as no more than 13, such as no more than 12, such as no more than 11, such as no more than 10, such as no more than 9, such as no more than 8, such as no more than 7 amino acid residues.

In some embodiments, the CPP is conjugated to a non-peptide moiety via its N-terminal. For example, the CPP may be methylated or acetylated. In some embodiments, when a peptide is defined herein to consist of a specific sequence of amino acid residues, said peptide is not conjugated to any other amino acid residue but said peptide may be conjugated to a non-peptide moiety, as long as the conjugation to a non-peptide moiety does not render another amino acid sequence. In some embodiments, the N-terminal of the CPP is acetylated i.e. bound to the chemical structure CH₃C(O)-. For example, the CPP may be a poly-Arg peptide consisting of nine L-arginine residues that is acetylated in the N-terminal. In some embodiments, the N-terminal of the CPP is conjugated to a chloroalkane tag (CA), which has the structure of:

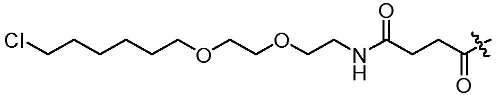

In one embodiment, the N-terminal of the CPP is methylated. In another embodiment, the N-terminal of the CPP is formylated.

### Linker

The term 'linker' as used herein refers to one or more atoms forming a connection from one chemical entity to another. By way of example, the 'linker' referred to herein may join the two PDZ-domain binding peptides P₁ and P₂ by forming a link to each of their N-termini. Various linkers are known in the art. The linker may for example be a chemical linker or a peptide linker, or a combination thereof. In one embodiment, the linker comprises an active functional group, such as an electrophilic or nucleophilic functional group, which can be used to attach the linker to each peptide.

In one embodiment, the linker comprises one or more polyethylene glycol (PEG) units. PEG is a polymer of ethylene glycol having the chemical formula C₂ₙH₄ₙ₊₂Oₙ₊₁, and the repeating structure: 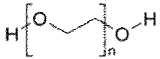 where n is an integer.

A PEG unit thus has the following structure:

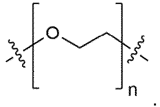

For example, a polymer consisting of 4 PEG moieties, or PEG4, corresponds to a polymer of 4 ethylene glycol moieties (n=4).

The terms "PEG unit" and "PEG moiety" are used interchangeably herein.

In one embodiment, at least one oxygen atom of one of the PEG units is replaced with a nitrogen atom to give *N*PEG. The term 'ethylene glycol moiety' as used herein refers to the structural unit that constitutes a PEG or *N*PEG linker.

In one embodiment, the N-termini of the two PDZ-domain binding peptides P₁ and P₂ are linked to each other via a linker comprising one or more PEG units, wherein at least one oxygen atom of the PEG units is optionally replaced with a nitrogen atom. In one embodiment, P₁ and/or P₂ are individually bound to the PEG/NPEG units via a spacer group, such as via a short alkane chain.

In one embodiment, the linker comprises one or more PEG units wherein at least one oxygen atom of one of the PEG units is replaced with a nitrogen atom to give NPEG.

In one embodiment, the linker comprises a NPEG unit and the CPP is linked to the linker via a chemical bond either directly or indirectly to the nitrogen atom in the backbone of the *N*PEG linker. Linkage of the CPP to the nitrogen of the *N*PEG linker may be mediated via an amide bond, a 1,3-dipolar cycloaddition such as copper catalyzed azide-alkyne cycloaddition, a maleimide coupling, a disulfide bond, or amino-reactive electrophilic groups, selected from among *N*-hydroxysuccinimide (NHS) ester, p-nitrophenyl ester, succinimidyl carbonate, p-nitrophenyl carbonate, succinimidyl urethane, isocyanate, isothiocyanate, acyl azide, sulfonyl chloride, aldehyde, carbonate, imidioester or anhydride; and thio-reactive groups selected from among haloacetyl, alkyl halide derivatives, aziridine, acryloyl derivatives arylating agents. In one embodiment, the linker comprises one or more PEG units wherein at least one oxygen atom of one of the PEG units is replaced with a nitrogen atom to give NPEG, and wherein the CPP is linked to the nitrogen atom of the linker by an amide bond. Alternatively, linkage of the CPP to the nitrogen of the linker comprising a *N*PEG unit may be mediated via a spacer group, where a suitable spacer group can for example be any amino acid(s); short alkane chains or short PEG/*N*PEG chains.

In one embodiment, the linker comprises a *N*PEG unit and the CPP is linked to the nitrogen atom of the linker by an amide bond. For example, in one embodiment, the compound has the general structure of Formula (III): 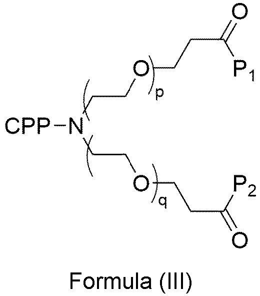 wherein
CPP, P₁ and P₂ are as defined herein;
p is an integer 0 to 10; and
q is an integer 0 to 10.

In one embodiment, p = q. In one embodiment, p > q. In one embodiment, p < q. In one embodiment, the sum of p and q is an integer between 1 and 20. In one embodiment, p is an integer selected from 0, 1, 2, 3, 4, 5, 6, 7, 8, 9 and 10. In one embodiment, p is an integer of 0 to 4. In one embodiment, q is an integer selected from 0, 1, 2, 3, 4, 5, 6, 7, 8, 9 and 10. In one embodiment, q is an integer of 0 to 4. In one embodiment, the total number of ethylene glycol moieties p + q is between 2 and 12, such as 2, such as 4, such as 6, such as 8, such as 10, such as 12. In one embodiment, the total number of ethylene glycol moieties p + q is 4. In one embodiment, p is 2 and q is 2.

### Overall dimeric structure

In one embodiment, the compound has the general structure of Formula (XXV): 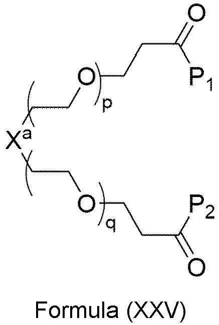 wherein
P₁ and P₂ are as defined herein;
X^{a} is 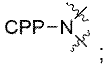
CPP is as defined herein;
p is an integer 0 to 10; and
q is an integer 0 to 10.

In one embodiment, p and q are 2, and the compound has the general structure of Formula (XXVI):

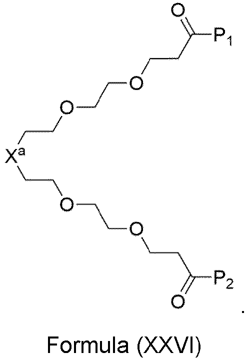

In one embodiment, the compound is of Formula (XXVI) wherein X^{a} is -O- and P₁ and P₂ are KETLV (SEQ ID NO: 2), thus forming compound 2, i.e. *O*PEG₄-(KETLV)₂.

In one embodiment, the compound is of Formula (XXVI) wherein X^{a} is -O- and P₁ and P₂ are KETTV (SEQ ID NO: 3), thus forming compound 3, i.e. *O*PEG₄-(KETTV)₂.

In one embodiment, the compound is of Formula (XXVI) wherein X^{a} is -O- and P₁ and P₂ are KETVV (SEQ ID NO: 4), thus forming compound 4, i.e. *O*PEG₄-(KETVV)₂.

In one embodiment, the compound is of Formula (XXVI) wherein X^{a} is -O- and P₁ and P₂ are KETRV (SEQ ID NO: 5), thus forming compound 5, i.e. *O*PEG₄-(KETRV)₂.

In one embodiment, the compound is of Formula (XXVI) wherein X^{a} is -O- and P₁ and P₂ are ISTDV (SEQ ID NO: 6), thus forming compound 6, i.e. *O*PEG₄-(ISTDV)₂.

In one embodiment, the compound is of Formula (XXVI) wherein X^{a} is -O- and P₁ and P₂ are VETVV (SEQ ID NO: 7), thus forming compound 7, i.e. *O*PEG₄-(VETVV)₂.

In one embodiment, the compound has the general structure of Formula (V): 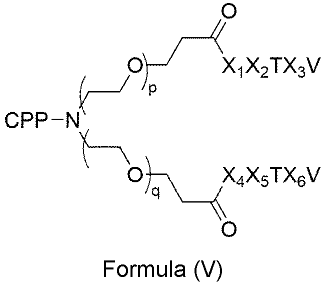 wherein
CPP is as defined herein;
X₁ is selected from the group consisting of K, V and I;
X₂ is selected from the group consisting of E and S; and
X₃ is selected from the group consisting of L, T, V, R and D.
X₄ is selected from the group consisting of K, V and I;
X₅ is selected from the group consisting of E and S;
X₆ is selected from the group consisting of L, T, V, R and D.
p is an integer 0 to 10; and
q is an integer 0 to 10.

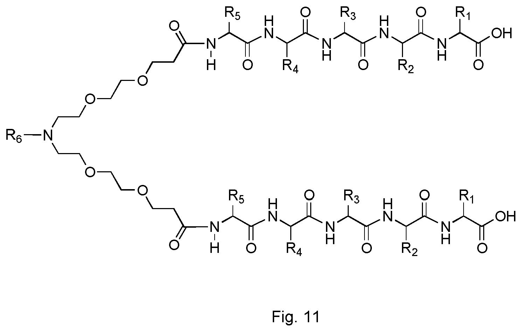

The compound of Formula (V) wherein p and q are 2 is further depicted in Figure 11. R₁ through R₅ are amino acid side-chains of the peptides X₁X₂TX₃V (SEQ ID NO: 54) and X₄X₅TX₆V (SEQ ID NO: 56), and R₆ represents a CPP-tag as described herein.

### Poly-Arg as CPP

In one embodiment, the CPP is a poly-L-arginine peptide (poly-Arg) consisting of 3 to 9 L-arginine residues. Thus, in one embodiment, the compound has the following general structure of Formula (II): 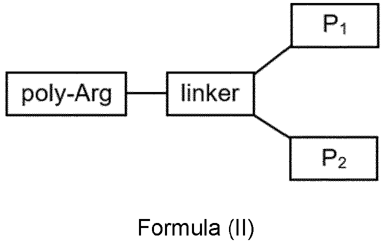 wherein poly-Arg, linker, P₁ and P₂ are as defined herein.

In one embodiment, the N-terminal of the poly-Arg is optionally modified, such as optionally acetylated.

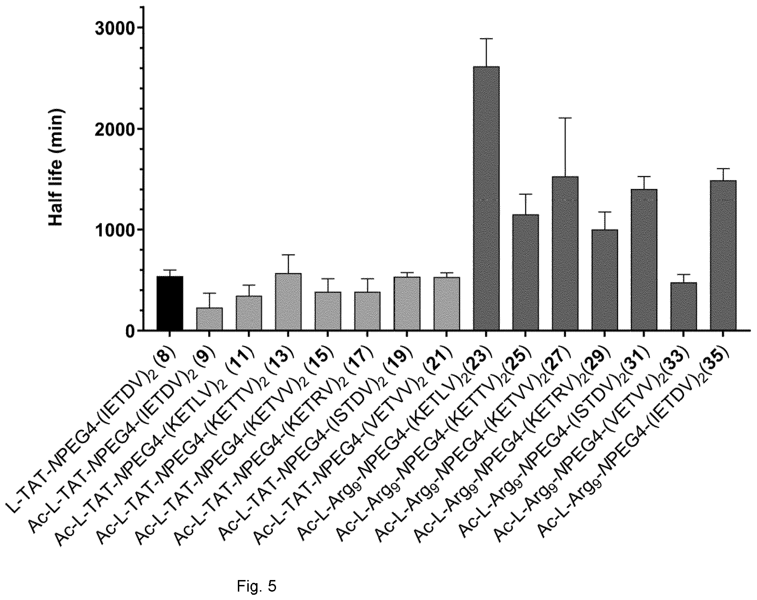

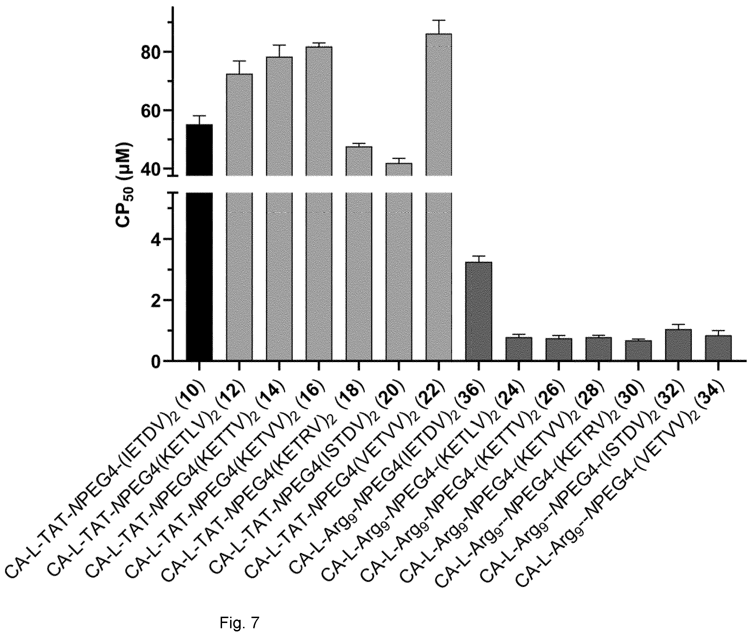

Compounds of the present invention with poly-Arg CPP-tags, such as L-Arg9 (SEQ ID NO: 12), have low CP₅₀ values (Example 4, Fig. 7-9), i.e. the cellular uptake is highly efficient, suggesting superior BBB penetration and intracellular delivery. Surprisingly, compounds containing the CPP L-Arg₉ (SEQ ID NO: 12) exhibited *in vitro* plasmin half-life times comparable to compounds containing D-amino acids or macrocyclic CPPs (Fig. 5-6). Interestingly, the half-life of L-Arg₉ containing compounds was significantly better than those of L-TAT (SEQ ID NO 8) (Fig. 5).

In one embodiment, the CPP is poly-Arg consisting of 3 to 9 L-arginine residues and the linker is a PEG linker comprising a *N*PEG unit, and the CPP is linked to the nitrogen atom of the linker by an amide bond. Thus, in one embodiment, the compound has the general structure of Formula (IV): 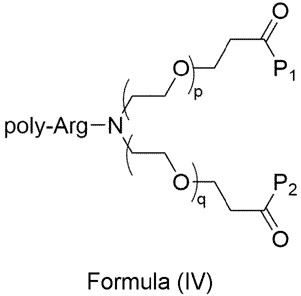 wherein
poly-Arg, P₁ and P₂ are as defined herein;
p is an integer 0 to 10; and
q is an integer 0 to 10.

In one embodiment, p is 2 and q is 2. Thus, in one embodiment, the compound has the general structure of Formula (VII): 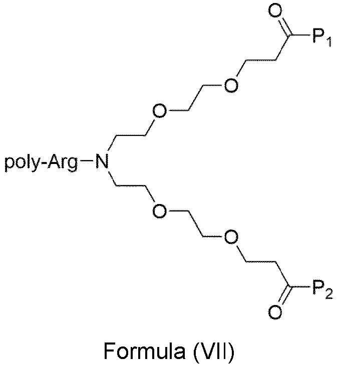 wherein poly-Arg, P₁ and P₂ are as defined herein.

In one embodiment, the compound has the general structure of Formula (VI): 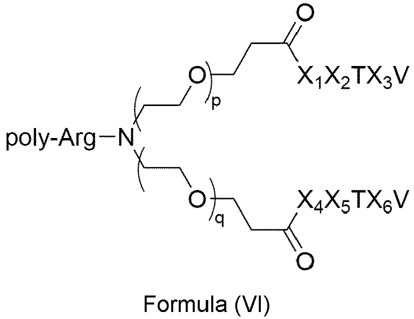 wherein
X₁ is selected from the group consisting of K, V and I;
X₂ is selected from the group consisting of E and S; and
X₃ is selected from the group consisting of L, T, V, R and D.
X₄ is selected from the group consisting of K, V and I;
X₅ is selected from the group consisting of E and S;
X₆ is selected from the group consisting of L, T, V, R and D.
p is an integer 0 to 10; and
q is an integer 0 to 10.

In one embodiment, p is 2 and q is 2. Thus, in one embodiment, the compound has the general structure of Formula (VIII): 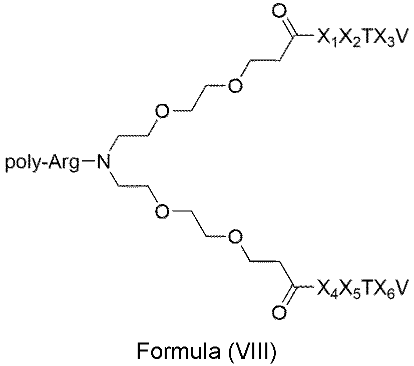 wherein poly-Arg, X₁, X₂ , X₃, X₄, X₅, and X₆ are as defined herein.

In one embodiment, the CPP is L-Arg₉, p is 2 and q is 2. Thus, in one embodiment, the compound has the general structure of Formula (IX): 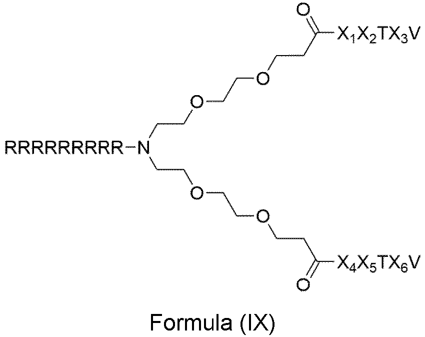 wherein X₁, X₂ , X₃, X₄, X₅, and X₆ are as defined herein.

In one embodiment, compound is selected from the group consisting of formulas (X) to (XVI):

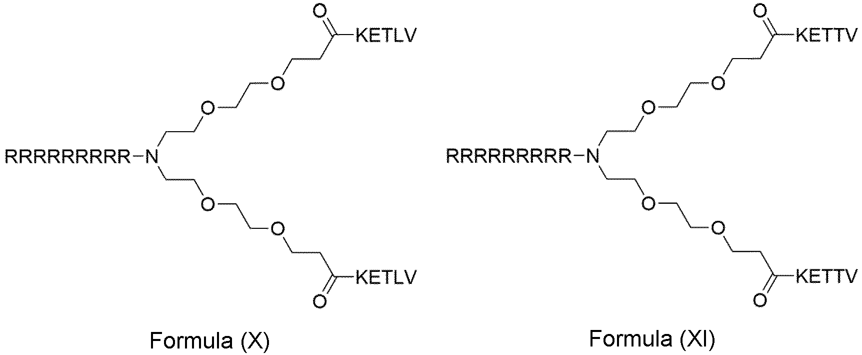

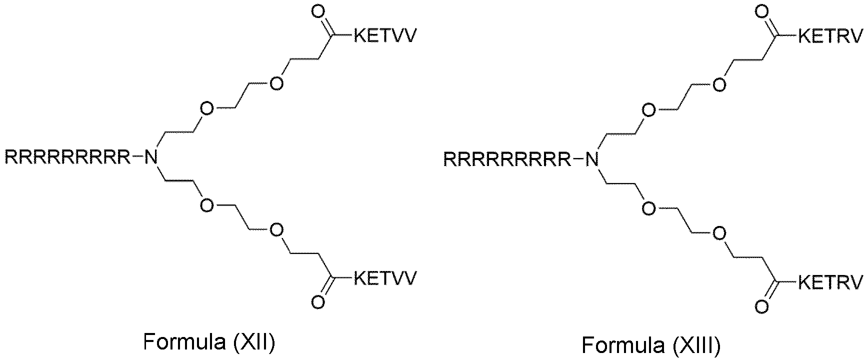

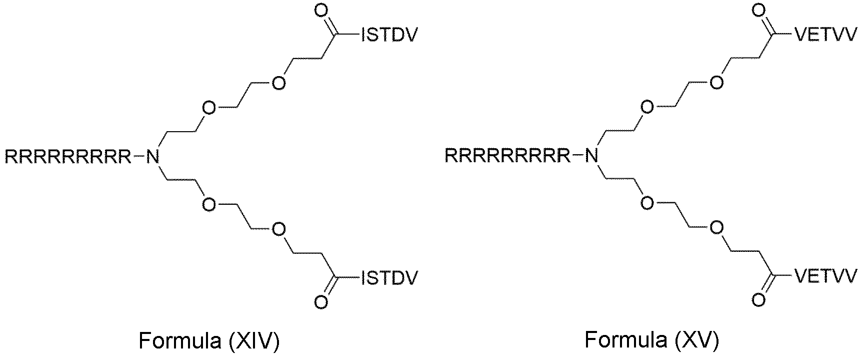

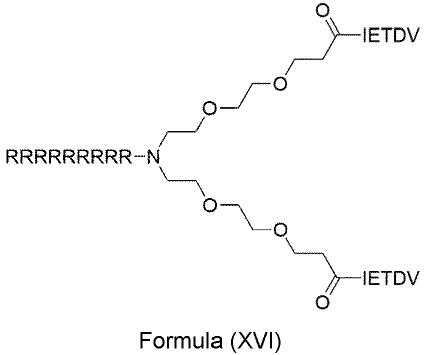

In one embodiment, any of the peptides P₁, P₂ and/or the CPP are further modified. For example, in one embodiment, the N-terminal of the CPP is optionally acetylated. Thus, in one embodiment, the compound is selected from the group consisting of formulas (XVII) to (XXIII):

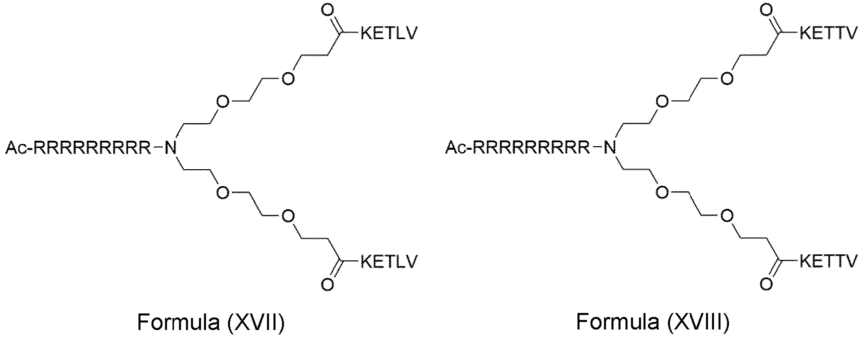

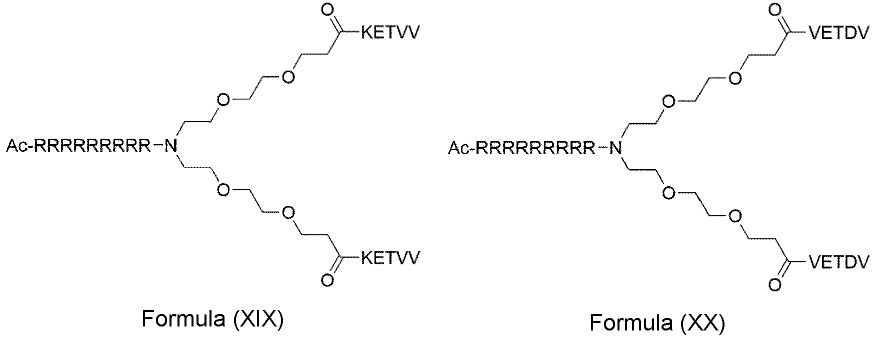

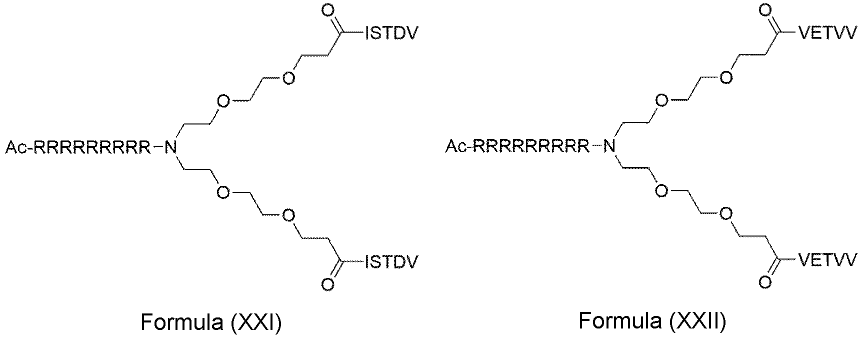

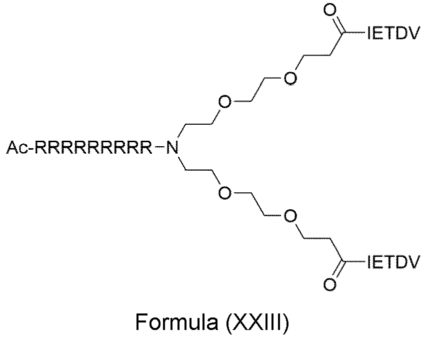

### Specific compounds

In one embodiment, the compound has the general structure of CPP- *N*PEG₄-(P₁)(P₂), i.e. the compound is of formula (XXVIII): 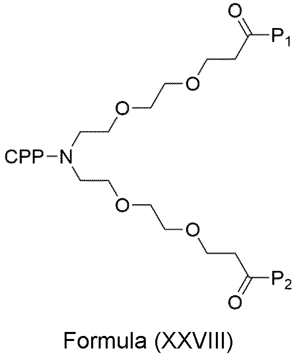 and CPP, P₁ and P₂ are as defined in table 2, wherein the compound is selected from the group consisting of compounds 23-39, 44-57, and 60-75.

**Table 2. Exemplified compounds of the invention.**

| **Compound** | **Formula** | **P₁, P₂** | **CPP** | |
|---|---|---|---|---|
| 8 *(AB144)* | | *IETDV (SEQ ID NO: 1)* | *L-TAT* | *YGRKKRRQRRR-(SEQ ID NO: 8)* |
| 9 | | IETDV (SEQ ID NO: 1) | L-TAT | Ac-YGRKKRRQRRR-(SEQ ID NO: 23) |
| 10 | | IETDV (SEQ ID NO: 1) | L-TAT | CA-YGRKKRRQRRR-(SEQ ID NO: 24) |
| 11 | | KETLV (SEQ ID NO: 2) | L-TAT | Ac-YGRKKRRQRRR-(SEQ ID NO: 23) |
| 12 | | KETLV (SEQ ID NO: 2) | L-TAT | CA-YGRKKRRQRRR-(SEQ ID NO: 24) |
| 13 | | KETTV (SEQ ID NO: 3) | L-TAT | Ac-YGRKKRRQRRR-(SEQ ID NO: 23) |
| 14 | | KETTV (SEQ ID NO: 3) | L-TAT | CA-YGRKKRRQRRR-(SEQ ID NO: 24) |
| 15 | | KETVV (SEQ ID NO: 4) | L-TAT | Ac-YGRKKRRQRRR-(SEQ ID NO: 23) |
| 16 | | KETVV (SEQ ID NO: 4) | L-TAT | CA-YGRKKRRQRRR-(SEQ ID NO: 24) |
| 17 | | KETRV (SEQ ID NO: 5) | L-TAT | Ac-YGRKKRRQRRR-(SEQ ID NO: 23) |
| 18 | | KETRV (SEQ ID NO: 5) | L-TAT | CA-YGRKKRRQRRR-(SEQ ID NO: 24) |
| 19 | | ISTDV (SEQ ID NO: 6) | L-TAT | Ac-YGRKKRRQRRR-(SEQ ID NO: 23) |
| 20 | | ISTDV (SEQ ID NO: 6) | L-TAT | CA-YGRKKRRQRRR-(SEQ ID NO: 24) |
| 21 | | VETVV (SEQ ID NO: 7) | L-TAT | Ac-YGRKKRRQRRR-(SEQ ID NO: 23) |
| 22 | | VETVV (SEQ ID NO: 7) | L-TAT | CA-YGRKKRRQRRR-(SEQ ID NO: 24) |
| 23 | (XVII) | KETLV (SEQ ID NO: 2) | L-Arg₉ | Ac-RRRRRRRRR-(SEQ ID NO: 25) |
| 24 | | KETLV (SEQ ID NO: 2) | L-Arg₉ | CA-RRRRRRRRR-(SEQ ID NO: 26) |
| 25 | (XVIII) | KETTV (SEQ ID NO: 3) | L-Arg₉ | Ac-RRRRRRRRR-(SEQ ID NO: 25) |
| 26 | | KETTV (SEQ ID NO: 3) | L-Arg₉ | CA-RRRRRRRRR-(SEQ ID NO: 26) |
| 27 | (XIX) | KETVV (SEQ ID NO: 4) | L-Arg₉ | Ac-RRRRRRRRR-(SEQ ID NO: 25) |
| 28 | | KETVV (SEQ ID NO: 4) | L-Arg₉ | CA-RRRRRRRRR-(SEQ ID NO: 26) |
| 29 | (XX) | KETRV (SEQ ID NO: 5) | L-Arg₉ | Ac-RRRRRRRRR-(SEQ ID NO: 25) |
| 30 | | KETRV (SEQ ID NO: 5) | L-Arg₉ | CA-RRRRRRRRR-(SEQ ID NO: 26) |
| 31 | (XXI) | ISTDV (SEQ ID NO: 6) | L-Arg₉ | Ac-RRRRRRRRR-(SEQ ID NO: 25) |
| 32 | | ISTDV (SEQ ID NO: 6) | L-Arg₉ | CA-RRRRRRRRR-(SEQ ID NO: 26) |
| 33 | (XXII) | VETVV (SEQ ID NO: 7) | L-Arg₉ | Ac-RRRRRRRRR-(SEQ ID NO: 23) |
| 34 | | VETVV (SEQ ID NO: 7) | L-Arg₉ | CA-RRRRRRRRR-(SEQ ID NO: 26) |
| 35 | (XXIII) | IETDV (SEQ ID NO: 1) | L-Arg₉ | Ac-RRRRRRRRR-(SEQ ID NO: 25) |
| 36 | | IETDV (SEQ ID NO: 1) | L-Arg₉ | CA-RRRRRRRRR-(SEQ ID NO: 26) |
| 38 | | IETDV (SEQ ID NO: 1) | D-TAT | Ac-ygrkkrrqrrr-(SEQ ID NO: 27) |
| 39 | | IETDV (SEQ ID NO: 1) | D-TAT | CA-ygrkkrrqrrr-(SEQ ID NO: 28) |
| 40 | | IETDV (SEQ ID NO: 1) | mTAT | Ac-rRrGrKkRr-(SEQ ID BO: 29) |
| 41 | | IETDV (SEQ ID NO: 1) | mTAT | CA-rRrGrKkRr-(SEQ ID NO: 30) |
| 42 | | IETDV (SEQ ID NO: 1) | riTAT | Ac-rrrqrrkkr-(SEQ ID NO: 31) |
| 43 | | IETDV (SEQ ID NO: 1) | riTAT | CA-rrrqrrkkr-(SEQ ID NO: 32) |
| 44 | | IETDV (SEQ ID NO: 1) | D-Arg₉ | Ac-rrrrrrrrr-(SEQ ID NO: 33) |
| 45 | | IETDV (SEQ ID NO: 1) | D-Arg₉ | CA-rrrrrrrrr-(SEQ ID NO: 34) |
| 46 | | IETDV (SEQ ID NO: 1) | L-Pen | Ac- RQIKIWFQNRRMKWKK- (SEQ ID NO: 35) |
| 47 | | IETDV (SEQ ID NO: 1) | L-Pen | CA- RQIKIWFQNRRMKWKK- (SEQ ID NO: 36) |
| 48 | | IETDV (SEQ ID NO: 1) | D-Pen | Ac-rqikiwfqnrrmkwkk-(SEQ ID NO: 37) |
| 49 | | IETDV (SEQ ID NO: 1) | D-Pen | CA-rqikiwfqnrrmkwkk-(SEQ ID NO: 38) |
| 50 | | IETDV (SEQ ID NO: 1) | L-DPV3 | Ac- RKKRRRESRKKRRRES- (SEQ ID NO: 39) |
| 51 | | IETDV (SEQ ID NO: 1) | L-DPV3 | CA- RKKRRRESRKKRRRES- (SEQ ID NO: 40) |
| 52 | | IETDV (SEQ ID NO: 1) | L-pVEC | Ac- LLIILRRRIRKQAHAHSK- (SEQ ID NO: 41) |
| 53 | | IETDV (SEQ ID NO: 1) | L-pVEC | CA- LLIILRRRIRKQAHAHSK- (SEQ ID NO: 42) |
| 54 | | IETDV (SEQ ID NO: 1) | L-MAP | Ac- KLALKLALKALKAALKLA- (SEQ ID NO: 43) |
| 55 | | IETDV (SEQ ID NO: 1) | L-MAP | CA- KLALKLALKALKAALKLA- (SEQ ID NO: 44) |
| 56 | | IETDV (SEQ ID NO: 1) | L-TP2 | Ac-PLIYLRLLRGQF-(SEQ ID NO: 45) |
| 57 | | IETDV (SEQ ID NO: 1) | L-TP2 | CA-PLIYLRLLRGQF-(SEQ ID NO: 46) |
| 58 | | IETDV (SEQ ID NO: 1) | D-TP2 | Ac-pliylrllrGqf-(SEQ ID NO: 47) |
| 59 | | IETDV | D-TP2 | CA-pliylrllrGqf- |
| | | (SEQ ID NO: 1) | | (SEQ ID NO: 48) |
| 60 | | IETDV (SEQ ID NO: 1) | MiniAp4 | Ac-\|(Dap)KAPETALD\|-(SEQ ID NO: 49) |
| 61 | | IETDV (SEQ ID NO: 1) | MiniAp4 | CA-\|(Dap)KAPETALD\|-(SEQ ID NO: 50) |
| 62 | | IETDV (SEQ ID NO: 1) | CPP12 | \|Ff(Nal2)RrRrQ\|GABA-K-(SEQ ID NO: 51) |
| 63 | | IETDV (SEQ ID NO: 1) | CPP12 | ;Ff(Nal2)RrRrQ;GABA- K(CA)- (SEQ ID NO: 52) |
| 64 | | IETDV (SEQ ID NO: 1) | L-Arg₇ | CA-RRRRRRR-(SEQ ID NO: 68) |
| 65 | | IETDV (SEQ ID NO: 1) | L-Arg₅ | CA-RRRRR-(SEQ ID NO: 70) |
| 66 | | IETDV (SEQ ID NO: 1) | L-Arg₃ | CA-RRR- |
| 67 | | KETLV (SEQ ID NO: 2) | L-Arg₇ | Ac-RRRRRRR-(SEQ ID NO: 63) |
| 68 | | KETLV (SEQ ID NO: 2) | L-Arg₇ | CA-RRRRRRR-(SEQ ID NO: 68) |
| 69 | | KETLV (SEQ ID NO: 2) | L-Arg₆ | Ac-RRRRRR-(SEQ ID NO: 64) |
| 70 | | KETLV (SEQ ID NO: 2) | L-Arg₉ | CA-RRRRRR-(SEQ ID NO: 69) |
| 71 | | KETLV (SEQ ID NO: 2) | L-Arg₅ | Ac-RRRRR-(SEQ ID NO: 65) |
| 72 | | KETLV (SEQ ID NO: 2) | L-Arg₅ | CA-RRRRR-(SEQ ID NO: 70) |
| 73 | | KETLV (SEQ ID NO: 2) | L-Arg₄ | Ac-RRRR-(SEQ ID NO: 66) |
| 74 | | KETLV (SEQ ID NO: 2) | L-Arg₄ | CA-RRRR-(SEQ ID NO: 71) |
| 75 | | KETLV (SEQ ID NO: 2) | L-Arg₃ | CA-RRR- |

In one embodiment, P₁ and P₂ consist of the amino acid sequence IETDV (SEQ ID NO: 1) and the CPP is selected from the group consisting of D-Pen (rqikiwfqnrrmkwkk, SEQ ID NO: 15); L-pVEC (LLIILRRRIRKQAHAHSK, SEQ ID NO: 18); L-TP2 (PLIYLRLLRGQF, SEQ ID NO: 19); MiniAp4 (|(Dap)KAPETALD|, SEQ ID NO: 21); and CPP12 (|Ff(Nal2)RrRrQ|GABA-K, SEQ ID NO: 22).

### Salts and prodrugs

The compound as defined herein can be in the form of a pharmaceutically acceptable salt or prodrug of said compound. In one embodiment of the present invention, the compound as defined herein is be formulated as a pharmaceutically acceptable addition salt or hydrate of said compound, such as but not limited to K⁺, Na⁺, as well as non-salt e.g. H⁺.

### Pharmaceutical composition

The compounds of the present invention can be comprised in a pharmaceutical composition. Formulation of a compound of the present invention into pharmaceutical compositions is well known in the art, and is further described in Gennaro (ed.), 2000, Remington: The Science and Practice of Pharmacy, 20th ed., Lippincott, Williams & Wilkins (2000); and Ansel et al., 1999, Pharmaceutical Dosage Forms and Drug Delivery Systems, 7th ed., Lippincott Williams & Wilkins Publishers.

### Membrane permeability

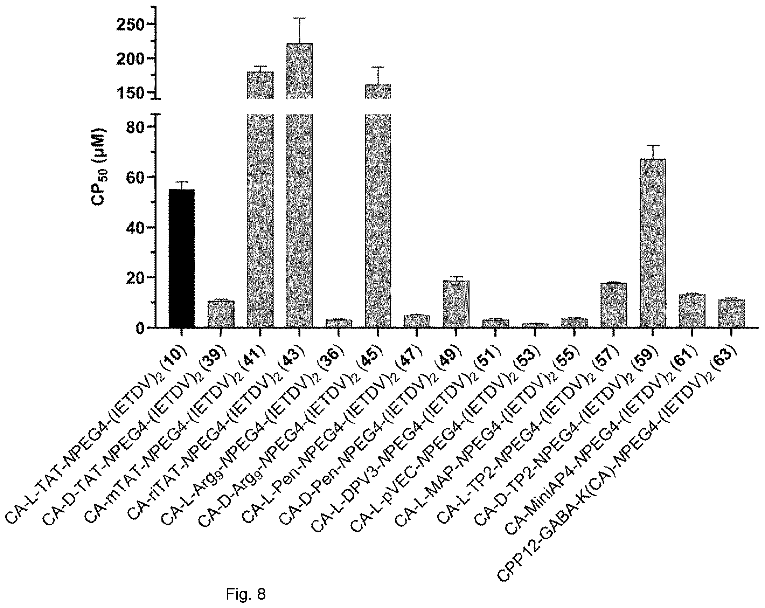

Since PSD-95 is located intracellularly, it is essential for any drug targeting PSD-95 to efficiently cross the cell membrane. To assess the cellular permeability and delivery to the cytosol of the compounds as defined herein, the cellular chloroalkane penetration assay (CAPA) may be used (Peraro et al. 2018). This assay takes advantage of a modified haloalkane dehalogenase designed to covalently bind chloroalkane (CA) molecules. A HeLa cell line expressing a fusion protein comprising a HaloTag, a green fluorescent protein (GFP) and a mitochondria-targeting peptide is used to report cytosolic delivery. The general format of the CAPA is a pulse-chase assay (Deprey & Kritzer, 2020). Cells expressing the HaloTag enzyme are incubated with CA-tagged peptides. When these CA-peptides penetrate the cell membrane and reach the cytosol, they will bind to and react with the HaloTag (*pulse step*)*.* Following a washing step, the cells are incubated with a CA-tagged dye that quantitatively penetrates the cell membrane and reacts with remaining unreacted HaloTag sites (*chase step*)*.* Flow cytometry is used to measure the fluorescence intensity of the cells and the measured fluorescence is inversely proportional to the amount of CA-peptides that reach the penetrated the cells and can thus be used to assess cytosolic delivery. The obtained data is commonly expressed as CP₅₀ values, the concentration at which 50% cell penetration is observed. The CP₅₀ value of a compound may be measured as described in example 4. Example 4 shows that the cellular uptake of described compounds greatly depend on the employed CPP, measuring CP₅₀ values ranging from 86.1 µM (compound 22) to 0.68 µM (compound 30) (Fig. 7). Further, the cellular uptake efficiency for *N*PEG₄(IETDV)₂ conjugated to a range of CPP-tags (SEQ ID NO 8 to 22) resulted in an unpredictable range of CP₅₀ values (Fig. 8).

In one embodiment, the compound has a CP₅₀ value of no more than 250 µM, such as no more than 200 µM, such as no more than 150 µM, such as no more than 100 µM, such as no more than 80 µM, such as no more than 70 µM, such as no more than 60 µM, such as no more than 50 µM, such as no more than 40 µM, such as no more than 30 µM, such as no more than 20 µM, such as no more than 15 µM, such as no more than 10 µM, such as no more than 5 µM. Preferably, the compound has a CP₅₀ value of no more than 60 µM.

### Plasmin stability

Ischemic stroke (also referred to as 'brain ischemia' or 'cerebral ischemia') is usually caused by a blockage in an artery that supplies blood to the brain. The blockage reduces the blood flow and oxygen to the brain, leading to damage or death of brain cells. The blockage of the blood vessels can be removed using a range of mechanical devices, or using "clot busting agents" which are delivered intravenously or intra-arterially. Among such clot busting agents is Tissue plasminogen factor (tPA), which generates plasmin from plasminogen. Examples of recombinant tPA's are alteplase, reteplase and tenecteplase, and other thrombolytic drugs that break down clots include streptokinase, urokinase and desmotaplase.

In one embodiment, the compounds of the present invention are administered to subjects receiving tPA or a recombinant tPA, which is the standard-of-care for AIS.

Thus, it is essential that the compound is compatible with the administration of tPA, including the generation of plasmin, which is a serine protease.

The *in vitro* plasmin stability of compounds of the present invention were determined in example 3. In one embodiment, the compound has a half-life in the plasmin stability assay described in example 3 of at least 10 min in the presence pf plasmin, such as at least 30 min, such as at least 1 h, such as at least 2 h, such as at least 3 h, such as at least 4 h, such as at least 5 h, such as at least 6 h, such as at least 7 h, such as at least 8 h, such as at least 9 h, such as at least 10 h, such as at least 15 h, such as at least 20 h, such as at least 30 h.

### Affinity to PDZ1-2 of PSD-95

Preferably, the compounds of the present invention have an affinity for the PDZ1-2 of PSD-95 in the nanomolar range, making them highly potent inhibitors. The affinity of the for the PDZ1-2 of PSD-95 is a critical factor in reducing the threshold concentration of drug needed to attain a therapeutic effect, which is particularly important when the drug must cross the blood brain barrier (BBB) to reach its target, since the BBB will tend to limit the accumulation of drug concentration at the target.

In some embodiments, the compound of the present invention is a dimeric PSD-95 inhibitor binding to PDZ1 and PDZ2 simultaneously, which may account for their high affinity for these domains.

As described in Example 2, the obtained affinities towards PDZ1-2 of PSD-95 for compounds of the present invention are in the low nanomolar range and similar to L-TAT-*N*PEG₄(IETDV)₂.

In one embodiment, the *Kᵢ* value for PDZ1-2 of PSD-95 of the compound is no more than 100 nM, such as no more than 80 nM, such as no more than 70 nM, such as no more than 60 nM, such as no more than 50 nM, such as no more than 40 nM, such as no more than 30 nM, such as no more than 20 nM, such as no more than 10 nM.

### Medical use

In one aspect the compound of the present invention as defined herein, is for use as a medicament.

The PDZ1 and PDZ2 domains of PSD-95 interact with several proteins including the simultaneous binding of the NMDA-type of ionotropic glutamate receptors and the nitric oxide (NO) producing enzyme nNOS. NMDA receptors are the principal mediators of excitotoxicity, i.e. glutamate-mediated neurotoxicity, which is implicated in neurodegenerative diseases and acute brain injuries. PSD-95 simultaneously binds the NMDA receptor, primarily GluN2A and GluN2B subunits, and nNOS via PDZ1 and PDZ2, respectively. Activation of the NMDA receptor causes influx of calcium ions, which activates nNOS thereby leading to NO generation. Thus, PSD-95 mediates a specific association between NMDA receptor activation and NO production, which can be detrimental for the cells if sustained for a longer period, and is a key facilitator of glutamate-mediated neurotoxicity. Inhibition of the ternary complex of nNOS/PSD-95/NMDA receptor interaction by targeting PSD-95 is known to prevent ischemic brain damage in mice, by impairing the functional link between calcium ion entry and NO production, while the physiological function, such as ion-flux and pro-survival signaling pathways of the NMDA receptor remains intact. Specific inhibition of excitotoxicity can be obtained by perturbing the intracellular nNOS/PSD-95/NMDA receptor complex using PSD-95 inhibitors.

The compounds of the present invention are PSD-95 inhibitors and are thus able to inhibit excitotoxicity. Hence, the compounds of the present invention are useful in treating a variety of diseases, particularly neurological diseases, and especially diseases mediated in part by excitotoxity. Such diseases and conditions include stroke, epilepsy, hypoxia, traumatic injury to the CNS not associated with stroke such as traumatic brain injury and spinal cord injury, other cerebral ischemia, Alzheimer's disease and Parkinson's disease.

In one aspect, the present invention relates to a compound of the invention for use in preventing, treating, reducing and/or delaying development of an excitotoxic-related disease. In one embodiment, the excitotoxic-related disease is stroke. In one embodiment, the excitotoxic-related disease is ischemic stroke. In one embodiment, the excitotoxic-related disease is cerebral ischemia. In one embodiment, the excitotoxic-related disease is acute ischemic stroke. In one embodiment, the excitotoxic-related disease is subarachnoid hemorrhage. In one embodiment, the excitotoxic-related disease is ischemic or traumatic injury to/in/of the CNS.

In one aspect, the present invention relates to a compound of the invention for use in reducing and/or protecting against a damaging effect of excitotoxicity. In one embodiment, the compound is for use in reducing the damaging effect of stroke. In one embodiment, the compound is for use in treating a damaging effect of acute ischemic stroke. In one embodiment, the compound is for use in treating a damaging effect of subarachnoid hemorrhage.

As used herein, "stroke" is a general term that refers to conditions caused by the occlusion or hemorrhage of one or more blood vessels supplying the brain, leading to cell death. "Ischemic stroke", as used herein, refers to stroke caused by an occlusion of one or more blood vessels supplying the brain. Types of ischemic stroke include, e.g., embolic stroke, cardioembolic stroke, thrombotic stroke, large vessel thrombosis, lacunar infarction, artery-artery stroke and cryptogenic stroke. "Cerebral ischemia" is a condition in which a blockage in an artery restricts the delivery of oxygen-rich blood to the brain, resulting in damage to brain tissue. Cerebral ischemia is sometimes called brain ischemia or cerebrovascular ischemia.

"Hemorrhagic stroke", as used herein, refers to stroke caused by hemorrhage of one or more blood vessels supplying the brain. Types of hemorrhagic stroke include, e.g., subdural stroke, intraparenchymal stroke, epidural stroke and subarachnoid stroke.

In one embodiment, the disease treatable by the compound of the present invention is ischemic or traumatic injury of the CNS

In one aspect, the present invention relates to a compound of the invention for use in inhibiting ischemic damage from neurosurgery. In one embodiment, said neurosurgery is diagnostic angiography of the brain or endovascular surgery to treat an aneurysm.

In some embodiments, the compound is administered in combination with reperfusion therapy. In one embodiment, the compound and the reperfusion are administered simultaneously, sequentially or separately to the subject.

The term 'reperfusion therapy' as used herein refers to a medical treatment to restore blood flow, either through or around, blocked arteries. Reperfusion therapy includes medical agents and mechanical reperfusion. Said medical agents may be thrombolytics or fibrinolytics used in a process called thrombolysis. In some embodiments, reperfusion therapy is performed by administering a thrombolytic agent, such as a plasminogen activator, for example tPA.

In one embodiment, the compound is compound 8 (AB144), and said compound is administered in combination with a plasminogen activator, for example tPA.

In some embodiments, the reperfusion therapy is mechanical reperfusion including surgery. Surgeries performed may be minimally-invasive endovascular procedures. Among mechanical reperfusion devices, there are intra-arterial catheters, balloons, stents, and various clot retrieval devices.

In one embodiment, the compound is administered in combination with a thrombolytic agent, and the compound and the thrombolytic agent are administered simultaneously, sequentially or separately to the subject.

In one aspect, the present invention relates to a compound of the invention for use in treating a damaging effect of ischemia on the central nervous system in a subject having or at risk of ischemia, wherein reperfusion therapy is performed on the subject, and the compound and reperfusion therapy treat a damaging effect of the ischemia on the central nervous system of the subject.

In one embodiment, the treatment further comprises administering a thrombolytic agent simultaneously, sequentially or separately to the subject.

In one aspect, the present invention relates to a kit of parts comprising at least two separate unit dosage forms (A) and (B), wherein
(A) comprises a compound of the invention; and
(B) comprises a thrombolytic agent.

In one aspect, the kit of parts as defined herein is for use in the treatment of a damaging effect of ischemia on the central nervous system, wherein (A) and (B) are administered simultaneously, sequentially or separately to the subject.

In one aspect, the present invention relates to a compound of the invention for use in treating a damaging effect of subarachnoid hemorrhage. The term "subarachnoid hemorrhage" as used herein refers to a hemorrhage state in a subarachnoid cavity.

Other neurological diseases treatable by the compounds of the present invention not known to be associated with excitotoxicity include anxiety and pain. Dimeric ligands targeting PSD-95 are under pre-clinical/clinical evaluation as a treatment for chronic pain and ischemic stroke (Andreasen et al., Neuropharmacol, 2013, 67, 193-200; Bach et al, PNAS USA, 2012, 109, 3317-3322).

In one embodiment, the subject as referred to herein is a mammal, such as a human.

### Synthesis

The compounds of the present invention may be manufactured by a method comprising the general steps of:
a) Synthesizing a peptide P₁/P₂ as defined herein;
b) Dimerizing the peptide of a) with an OPEGₙ or *N*PEGₙ linker, wherein 'n' is the number of PEG moieties;
c) Attaching a CPP tag at the N of the NPEG linker, for example by using an automated peptide synthesizer.

In one embodiment, the compounds are manufactured by a method comprising the general steps of:
a) providing a Ns-NPEG diacid linker;
b) preparing a peptide P₁/P₂ using Fmoc-based solid-phase peptide synthesis;
c) dimerizing Fmoc-deprotected peptide P₁/P₂ with Ns-NPEG diacid linker forming a linker-dimer conjugate; and
d) attaching a CPP to the N of the *N*PEG linker of the linker-dimer conjugate, for example by using an automated peptide synthesizer.

In one embodiment, the compounds of the present invention are synthesized as described below.

### Ns-NPEG diacid linker:

The 'Ns-*N*PEG diacid linker' is the structure where an NPEG linker is protected on the nitrogen with an *ortho*-nitrobenzenesulfonyl (Ns) protection group on the linker nitrogen, and where the termini of the *N*PEG linker comprise carboxylic acids. This chemical reagent or building block is used to dimerize the two peptide moieties, P₁ and P₂.

The ortho-nitrobenzenesulfonyl (Ns)-protected *N*PEG linker is produced either on solid-phase or in solution.

The solid-phase procedure typically starts by loading a solid support useful for solid-phase peptide synthesis, such as 2-chlorotrityl chloride resin, with Fmoc-NH-PEG-CH₂CH₂COOH, using appropriate organic solvent for the specific resin (e.g. DCM, DMF**,** ACN, THF) and a base (e.g. DIPEA, DBU, collidine, NMM)

The Fmoc group can be removed by base (e.g. piperidine, dimethylamine, morpholine, piperazine, dicyclohexylamine, DMAP) in appropriate solvent (e.g. DMF**,** DCM, ACN, THF).

Ortho-nitrobenzenesulfonyl chloride can be coupled to the free amine using base (e.g. DIPEA, DBU, collidine, NMM) and appropriate solvent (e.g. THF**,** DCM) to get Ns-NH-PEG-CH₂CH₂COO-Resin.

The second part of the linker product can be connected to the resin-bound linker-part by the use of Mitsunobu-chemistry. Resin is treated with triphenylphosphine, HO-PEG-CH₂CH₂COO*t*Bu, solvent, and ester- or amide reagents of azodicarboxylic acid (e.g. diisopropyl azodicarboxylate, DIAD; diethyl azodicarboxylate, DEAD; 1,1'-(Azodicarbonyl)-dipiperidine, ADDP).

The final Ns-*N*PEG diacid linker is obtained by treating the resin with acid, such as trifluoroacetic acid (TFA).

The solution-phase procedure can be performed by protection of the amine group of NH₂-PEG-CH₂CH₂COO*t*Bu with Ns, followed by Mitsunobu chemistry in solution using triphenylphosphine and DIAD, DEAD, or ADDP, or similar reagents, HO-PEG-CH₂CH₂COO*t*Bu, and appropriate solvent (THF, DCM). Final Ns-protected *N*PEG-linker is then obtained by treatment with acids, such as TFA.

Peptide synthesis: The peptide sequence is synthesized by Fmoc-based solid-phase peptide synthesis using a solid support, such as 2-chlorotrityl chloride resin or Wang resin, Fmoc-protected amino acids, base, coupling reagents (e.g. HBTU [*N,N,N',N'-*Tetramethyl-*O*-(1*H*-benzotriazol-1-yl)uronium hexafluorophosphate], *O*-(7-azabenzotriazol-1-yl)-*N*,*N*,*N*',*N*'-tetramethyluronium hexafluorophosphate [HATU], PyBOB, DIC/HOBt) and solvents. Alternatively, to coupling reagents, activated ester of Fmoc-protected amino acids (e.g. pentafluorophenyl, succinimide) can be used.

Dimerization: The Fmoc-deprotected resin-bound peptide is dimerized with the Ns-NPEG diacid linker by an on-resin dimerization process by repetitive treatments of the resin with the Ns-*N*PEG diacid linker in sub-stoichiometric amounts (e.g. 1/6), base, coupling reagent, and appropriate solvents (e.g. DMF, DCM, THF). Alternatively to coupling reagents, activated ester of the Ns-NPEG linker can be used.

The dimerization process can also be formed in solution using either the activated ester (e.g. pentafluorophenyl, succinimide) of the Ns-NPEG linker together with 1-Hydroxy-7-azabenzotriazole (HOAt) or Hydroxybenzotriazole (HOBt) and appropriate side chain-protected peptide (e.g. tert-butyl) in solvent (e.g. ACN, DMF, DCM, THF). Also, dimerization in solution can be performed using the Ns-*N*PEG diacid linker, coupling reagents (e.g. HBTU, HATU etc), base and solvents.

The Ns-group is removed by mercaptoethanol and 1,8-diazabicyclo[5.4.0]undec-7-ene (DBU) or by sodium thiophenolate.

Attachment of a CPP to the NH moiety of the *N*PEG linker of the linker-dimer conjugate: First, an amino acid is coupled to the NH moiety of the NPEG linker, and subsequently the CPP is synthesized following standard (manual/automated) Fmoc SPPS on resin.

Ester protection groups can be removed by stirring the cleaved products in aqueous base (e.g. NaOH, LiOH) and acetonitrile followed by acidification with TFA or HCl.

The final compound of the present invention is obtained by lyophilization and purification by HPLC or similar chromatographic methods.

In a further embodiment, the synthesis of the compounds of the present invention is performed as outlined in example 1.

### Examples

### Example 1: Synthesis

The resin bound compounds **2** to **7** (*O*PEG₄-KETLV , *O*PEG₄-KETTV, *O*PEG₄-KETVV, *O*PEG₄-KETRV, *O*PEG₄-ISTDV and *O*PEG₄-VETVV) were synthesized as previously described: Bach et al., Angew. Chem. Int. Ed., 2009, 48, 9685.

The resin bound *N*PEG₄-IETDV, *N*PEG₄-KETLV, *N*PEG₄-KETTV, *N*PEG₄-KETVV, *N*PEG₄-KETRV, *N*PEG₄-ISTDV and *N*PEG₄-VETVV were synthesized as previously described by Bach et al, Proc. Natl. Acad. Sci. USA, 2012, 109, 3317. The first C-terminus amino acid (Fmoc-Ala-OH, Fmoc-L-Arg(Pbf)-OH, Fmoc-D-Arg(Pbf)-OH, Fmoc-Asp(OAII)-OH, Fmoc-L-Lys(Boc)-OH, Fmoc-D-Lys(Boc)-OH, Fmoc-L-Lys(Ns)-OH, Fmoc-L-Phe-OH, Fmoc-D-Phe-OH, , Fmoc-L-Ser(tBu)-OH) of the linear CPPs (L-TAT, DTAT, mTAT, riTAT, polyR, D-polyR, L-Pen, D-Pen, L-pVEC, L-MAP, L-DPV3, L-TP2, D-TP2, MiniAp4, CPP12) was coupled manually to the nitrogen of the *N*PEG4 linker three times using O-(1*H*-6-chlorobenzotriazole-1-yl)-1,1,3,3- tetramethyluronium hexafluorophosphate (HCTU) as coupling reagent. Subsequent, synthesis of the CPPs was achieved using an automated peptide synthesizer (Prelude X, Gyros Protein Technologies, US). For the synthesis using Prelude X all reagents were prepared as solutions in DMF: Fmoc- protected amino acids (0.2 M), HCTU (0.4 M), and DIPEA (1.0 M). Sequence elongation was achieved using the following protocol: deprotection (2 × 2 min, rt, 350 rpm shaking) and coupling (3 × 10 min, 50 °C, 350 rpm shaking). Amino acids were triple coupled using a mixture of Fmoc-amino acid/HCTU/DIPEA (1:1:2.5) in 5-fold excess over the resin loading. The cyclic CPP (MiniAp4 and CPP12) containing peptides were treated with 20 eq. PhSiH₃ and 0.2 eq. Pd(PPh₃)₄ in dichloromethane (DCM) under nitrogen for 2 × 15 min to remove allyl and alloc protecting groups. The resin was washed with DCM followed by dimethylformamide (DMF). The cyclization reaction between the side chains of Asp/Dap and or C-terminus of Glu and N-terminus of Phe was performed by treatment of the peptide with 2 eq. (benzotriazol-1-yloxy)tripyrrolidinophosphonium hexafluorophosphate (PyBOP) and 2 eq. DIPEA in DMF for 16 h at room temperature followed by DMF wash. After successful synthesis of CPP tagged dimeric peptides, the resin was split into two. The N-terminus of one part of resin-bound peptides was capped using a mixture of DMF:acetic anhydride:DIPEA (8:1.5:0.5) twice for 10 min. The N-terminus of other part of the resin-bound peptides was functionalized with chloroalkane tag (CA). The CA tag was coupled to the nitrogen group of the N-terminus of the CPP tagged peptides using a mixture of CA:PyBOP:DIPEA in DMF (3:3:10) for 16 h. In case of CPP12 containing peptide, prior to CA tag coupling the ortho-nitrobenzenesulfonyl (Ns) group was removed by adding 1,8-diazabicyclo[5.4.0]undec-7-ene (DBU, 0.5 mmol) in DMF (2 mL) and mercaptoethanol (0.5 mmol) in DMF (2 mL) and shaking for 30 min. After a flow-wash in DMF, the mercaptoethanol/DBU treatment was repeated four times. After a final flow-wash in DMF, the CA tag was coupled to the side chain nitrogen of Lys residue.

The peptide compounds **2** to **75** were cleaved from dried resin with TFA/triisopropylsilane/ 2,2'-(ethylenedioxy)diethanethiol(DODT)/H₂O (90/2.5/2.5) for 2 h followed by filtration, evaporation, precipitation with ice-cold ether, lyophilization, and purification by preparative RP-HPLC. The final peptide ligands were characterised by LC-MS for molecular weight determination and UPLC (214 nm) for purity (>95%).

The synthesis, as described above, can also be conducted at 40 mmol or up to 100 mmol scale resulting multi-gram preparation of the peptide compounds reported herein.

The peptide compounds described herein exhibited excelled solubility properties reaching 25 mg/mL concentrations and higher.

This example demonstrates that the ligands can be synthesized, purified and obtained in pure form.

### Example 2: Determination of affinity to PDZ1-2 of PSD-95

The binding affinity of the compounds **1** to **75** to PDZ1-2 of PSD-95 was measured using an *in vitro* fluorescent polarization (FP) assay as described by Bach et al., Proc. Natl. Acad. Sci. USA, 2012, 109, 3317. First, a saturation binding curve was obtained to determine *K_{D}* values for the interaction between a fluorescent dimeric probe and PDZ1-2 of PSD-95. Increasing concentrations of PDZ1-2 (0.015-30 nM) were added to a constant concentration of the probe (0.5 nM). The fluorescence polarization (FP) of the sample was measured at excitation/emission wavelength of 635/670 nm and the generated FP values were fitted to a one site binding model using the software GraphPad Prism. Then, the affinity of non-labeled peptides to PSD-95 PDZ12 was evaluated by a heterologous competition binding assay in which tested peptides were added in increasing concentrations (0.4-960 nM, final concentration) to a fixed amount of PSD-95 PDZ12 (4 nM) and probe (0.5 nM) using the same conditions as for the saturation binding experiment. The FP values were fitted to a one site competition (variable slope) model in GraphPad prism. The resulting IC₅₀ values were converted to *Kᵢ* values as described previously by Nikolovska-Coleska et al., Anal. Biochem., 2004, 332, 261).

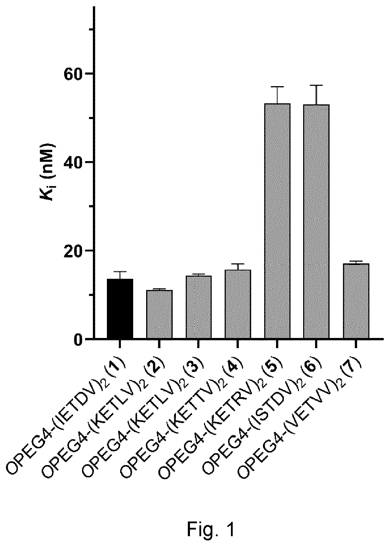

This example describes how to determine the affinity of ligands binding to PDZ1-2 of PSD-95. The results are presented in tables 4 to 7, and Figures 1 to 4. In conclusion, the tested compounds exhibited affinities (*Kᵢ* values) in the low nanomolar range which are comparable to compound 1.

**Table 4. Binding affinities (Kᵢ), presented as mean ± SEM (Figure 1).**

| **Compound** | **Ligand** | **CPP** | **CPP sequence** | ***K*ᵢ [nM]** |
|---|---|---|---|---|
| **1** | *O*PEG₄-(IETDV)₂ | - | - | 13.7 ± 1,6 |
| **2** | *O*PEG₄-(KETLV)₂ | - | - | 11.1 ± 0,3 |
| **3** | *O*PEG₄-(KETTV)₂ | - | - | 14.4 ± 0,4 |
| **4** | *O*PEG₄-(KETVV)₂ | - | - | 15.8 ± 1,2 |
| **5** | *O*PEG₄-(KETRV)₂ | - | - | 53.2 ± 3,8 |
| **6** | *O*PEG₄-(ISTDV)₂ | - | - | 53.1 ± 4,4 |
| **7** | *O*PEG₄-(VETVV)₂ | - | - | 17.0 ± 0,6 |

| | | | | |
|---|---|---|---|---|
| Data represented as n ≥ 3. | | | | |

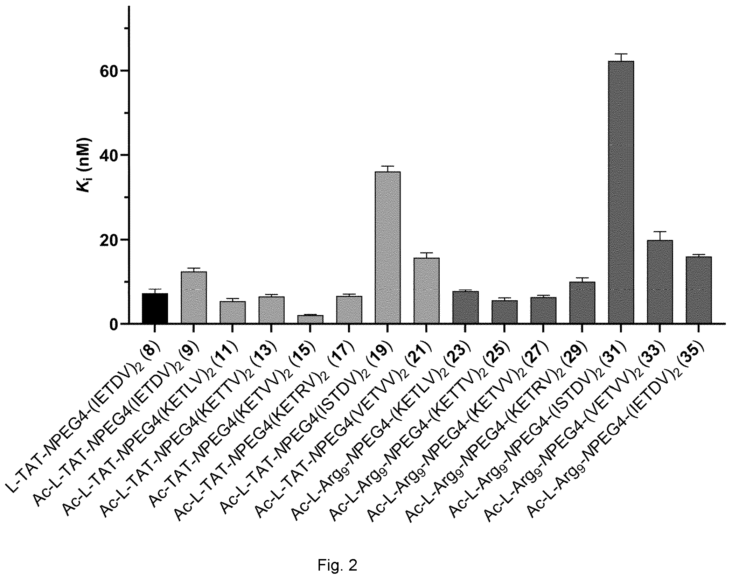

**Table 5. Binding affinities (Kᵢ), presented as mean ± SEM, for dimeric ligands fused to L-TAT and L-Arg₉ (Figure 2).**

| **Compound** | **Ligand** | **CPP** | **CPP sequence** | ***K*ᵢ [nM]** |
|---|---|---|---|---|
| **8** | *N*PEG₄-(IETDV)₂ | L-TAT | YGRKKRRQRRR- | 7.2 ± 1,5 |
| **9** | *N*PEG₄-(IETDV)₂ | L-TAT | Ac-YGRKKRRQRRR- | 12.5 ± 1,1 |
| **11** | *N*PEG₄-(KETLV)₂ | L-TAT | Ac-YGRKKRRQRRR- | 5.4 ± 0,9 |
| **13** | *N*PEG₄-(KETTV)₂ | L-TAT | Ac-YGRKKRRQRRR- | 6.5 ± 0,7 |
| **15** | *N*PEG₄-(KETVV)₂ | L-TAT | Ac-YGRKKRRQRRR- | 2.1 ± 0,2 |
| **17** | *N*PEG₄-(KETRV)₂ | L-TAT | Ac-YGRKKRRQRRR- | 6.6 ± 0,6 |
| **19** | *N*PEG₄-(ISTDV)₂ | L-TAT | Ac-YGRKKRRQRRR- | 36.1 ± 1,8 |
| **21** | *N*PEG₄-(VETVV)₂ | L-TAT | Ac-YGRKKRRQRRR- | 15.8 ± 1,6 |
| **35** | *N*PEG₄-(IETDV)₂ | L-Arg₉ | Ac-RRRRRRRRR- | 16.0 ± 0,6 |
| **23** | *N*PEG₄-(KETLV)₂ | L-Arg₉ | Ac-RRRRRRRRR- | 7.8 ± 0,4 |
| **25** | *N*PEG₄-(KETTV)₂ | L-Arg₉ | Ac-RRRRRRRRR- | 5.6 ± 0,8 |
| **27** | *N*PEG₄-(KETVV)₂ | L-Arg₉ | Ac-RRRRRRRRR- | 6.3 ± 0,7 |
| **29** | *N*PEG₄-(KETRV)₂ | L-Arg₉ | Ac-RRRRRRRRR- | 10.1 ± 1,3 |
| **31** | *N*PEG₄-(ISTDV)₂ | L-Arg₉ | Ac-RRRRRRRRR- | 62.3 ± 2,4 |
| **33** | *N*PEG₄-(VETVV)₂ | L-Arg₉ | Ac-RRRRRRRRR- | 19.9 ± 2,8 |

| | | | | |
|---|---|---|---|---|
| Data represented as n ≥ 3; Ac = *N*-terminal acetylation. | | | | |

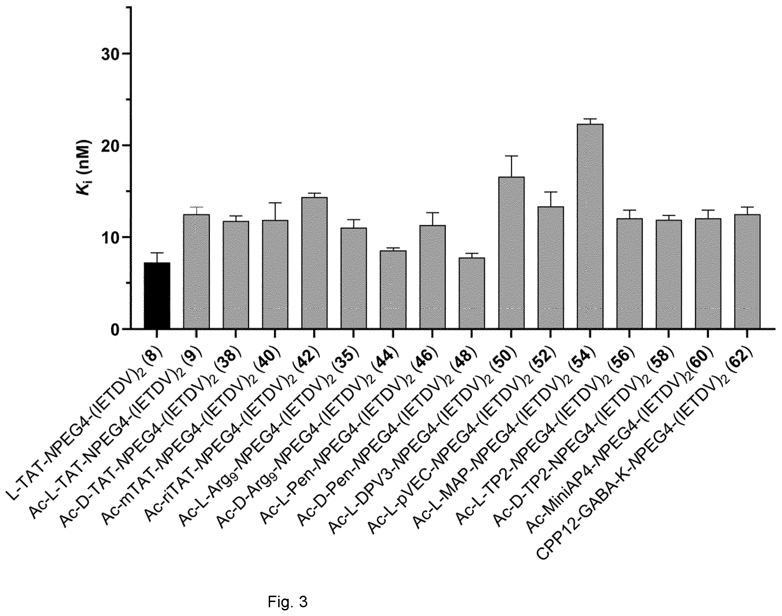

**Table 6. Binding affinities (Kᵢ), presented as mean ± SEM, for NPEG₄-(IETDV)₂ dimeric ligands fused to various CPP-tags (Figure 3).**

| **Compound** | **Ligand** | **CPP** | **CPP sequence** | ***K*ᵢ [nM]** |
|---|---|---|---|---|
| **8** | *N*PEG₄-(IETDV)₂ | L-TAT | YGRKKRRQRRR- | 7.2 ± 1,5 |
| **9** | *N*PEG₄-(IETDV)₂ | L-TAT | Ac-YGRKKRRQRRR- | 12.5 ± 1,1 |
| **38** | *N*PEG₄-(IETDV)₂ | D-TAT | Ac-yGrkkrrqrrr- | 11.8 ± 0,8 |
| **40** | *N*PEG₄-(IETDV)₂ | mTAT | Ac-rRrGrKkRr- | 11.9 ± 2,7 |
| **42** | *N*PEG₄-(IETDV)₂ | riTAT | Ac-rrrqrrkkr- | 14.4 ± 0,6 |
| **35** | *N*PEG₄-(IETDV)₂ | L-Arg₉ | Ac-RRRRRRRRR- | 11.0 ± 1,3 |
| **44** | *N*PEG₄-(IETDV)₂ | D-Arg₉ | Ac-rrrrrrrrr- | 8.6 ± 0,4 |
| **46** | *N*PEG₄-(IETDV)₂ | L-Pen | Ac- RQIKIWFQNRRMKWKK- | 11.3 ± 1,9 |
| **48** | *N*PEG₄-(IETDV)₂ | D-Pen | Ac-rqikiwfqnrrmkwkk- | 7.8 ± 0,7 |
| **50** | *N*PEG₄-(IETDV)₂ | L-DPV3 | Ac- RKKRRRESRKKRRRES- | 16.6 ± 3,2 |
| **52** | *N*PEG₄-(IETDV)₂ | L-pVEC | Ac- LLIILRRRIRKQAHAHSK- | 13.3 ± 2,2 |
| **54** | *N*PEG₄-(IETDV)₂ | L-MAP | Ac- KLALKLALKALKAALKLA- | 22.4 ± 0,8 |
| **56** | *N*PEG₄-(IETDV)₂ | L-TP2 | Ac-PLIYLRLLRGQF- | 12.1 ± 1,3 |
| **58** | *N*PEG₄-(IETDV)₂ | D-TP2 | Ac-pliylrllrGqf- | 11.9 ± 0,7 |
| **60** | *N*PEG₄-(IETDV)₂ | MiniAp4 | Ac-\|(Dap)KAPETALD\|- | 12.1 ± 1,3 |
| **62** | *N*PEG₄-(IETDV)₂ | CPP12 | \|Ff(Nal2)RrRrQ\|GABA-K- | 12.5 ± 1,1 |

| | | | | |
|---|---|---|---|---|
| Data represented as n ≥ 3; Ac = *N*-terminal acetylation. | | | | |

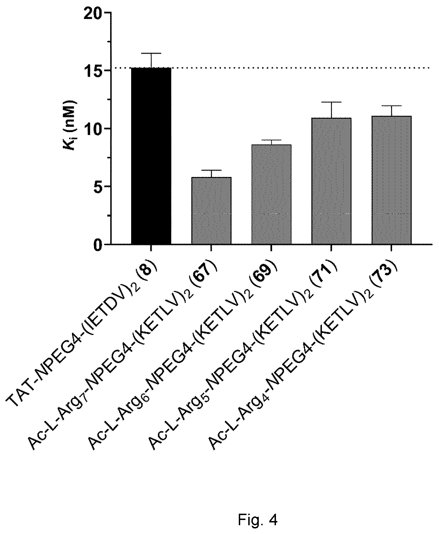

**Table 7. Binding affinities (Kᵢ), presented as mean ± SEM, for NPEG₄-(KETLV)₂ dimeric ligands fused to polyArg CPP-tags (Figure 4).**

| **Compound** | **Ligand** | **CPP** | **CPP sequence** | ***K*ᵢ [nM]** |
|---|---|---|---|---|
| **67** | *N*PEG₄-(KETLV)₂ | L-Arg₇ | Ac-RRRRRRR- | 5.8 ± 0,6 |
| **69** | *N*PEG₄-(KETLV)₂ | L-Arg₉ | Ac-RRRRRR- | 8.6 ± 0,3 |
| **71** | *N*PEG₄-(KETLV)₂ | L-Arg₅ | Ac-RRRRR- | 10.9 ± 1,3 |
| **72** | *N*PEG₄-(KETLV)₂ | L-Arg₄ | Ac-RRRR- | 11.1 ± 0,9 |

| | | | | |
|---|---|---|---|---|
| Data represented as n ≥ 3; Ac = *N*-terminal acetylation. | | | | |

### Example 3: In vitro plasmin stability of ligands

The *in vitro* plasmin stability of the compounds **8** to **62** was determined by incubating 100 µM of ligand in phosphate buffered saline (PBS) supplemented with plasmin (5 ug/mL) at 37 °C for 0-1440 minutes. At selected time points during the incubation, the ligands were extracted from 80 µL of assay matrix by treatment with 80 µL of 50% acetonitrile (ACN). The samples were filtered and analysed by UPLC to determine the amount of ligand remaining. LC-MS analysis was performed to confirm ligand integrity and identify cleavage sites.

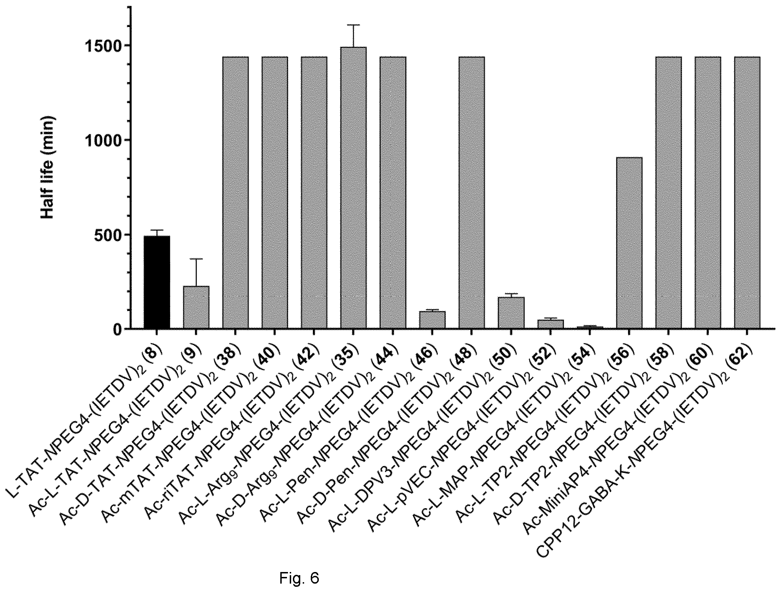

This example describes how to determine the *in vitro* plasmin stability of ligands binding to PDZ1-2 of PSD-95. The results are presented in tables 8 and 9, and Figures 5 and 6. In conclusion, the tested compounds containing the CPP L-Arg₉ (SEQ ID NO 12) exhibit *in vitro* plasmin half-life times comparable to compounds containing D-amino acids or macrocyclic CPPs (Fig. 6). Unexpectedly, the half-life of L-Arg₉ containing compounds was significantly better than those of L-TAT (SEQ ID NO 8) (Fig. 5, and 6).

**Table 8. In vitro plasmin stability, half-life times presented as mean ± SEM, for NPEG₄ dimeric ligands fused to L-TAT and L-Arg₉ (Figure 5).**

| **Compound** | **Ligand** | **CPP** | **CPP sequence** | **T_{1/2} [min]** |
|---|---|---|---|---|
| **8** | *N*PEG₄-(IETDV)₂ | L-TAT | YGRKKRRQRRR- | 543 ± 60 |
| **9** | *N*PEG₄-(IETDV)₂ | L-TAT | Ac-YGRKKRRQRRR- | 229 ± 129 |
| **11** | *N*PEG₄-(KETLV)₂ | L-TAT | Ac-YGRKKRRQRRR- | 346 ± 105 |
| **13** | *N*PEG₄-(KETTV)₂ | L-TAT | Ac-YGRKKRRQRRR- | 570 ± 180 |
| **15** | *N*PEG₄-(KETVV)₂ | L-TAT | Ac-YGRKKRRQRRR- | 385 ± 130 |
| **17** | *N*PEG₄-(KETRV)₂ | L-TAT | Ac-YGRKKRRQRRR- | 385 ± 130 |
| **19** | *N*PEG₄-(ISTDV)₂ | L-TAT | Ac-YGRKKRRQRRR- | 534 ± 40 |
| **21** | *N*PEG₄-(VETVV)₂ | L-TAT | Ac-YGRKKRRQRRR- | 532 ± 41 |
| **35** | *N*PEG₄-(IETDV)₂ | L-Arg₉ | Ac-RRRRRRRRR- | 1492 ± 114 |
| **23** | *N*PEG₄-(KETLV)₂ | L-Arg₉ | Ac-RRRRRRRRR- | 2618 ± 276 |
| **25** | *N*PEG₄-(KETTV)₂ | L-Arg₉ | Ac-RRRRRRRRR- | 1152 ± 203 |
| **27** | *N*PEG₄-(KETVV)₂ | L-Arg₉ | Ac-RRRRRRRRR- | 1530 ± 578 |
| **29** | *N*PEG₄-(KETRV)₂ | L-Arg₉ | Ac-RRRRRRRRR- | 1003 ± 171 |
| **31** | *N*PEG₄-(ISTDV)₂ | L-Arg₉ | Ac-RRRRRRRRR- | 1405 ± 124 |
| **33** | *N*PEG₄-(VETVV)₂ | L-Arg₉ | Ac-RRRRRRRRR- | 479 ± 77 |

| | | | | |
|---|---|---|---|---|
| Data represented as n ≥ 3; Ac = *N*-terminal acetylation. | | | | |

**Table 9. In vitro plasmin stability, half-life times presented as mean ± SEM, for NPEG₄-(IETDV)₂ dimeric ligands fused to various CPP-tags (Figure 6).**

| **Compound** | **Ligand** | **CPP** | **CPP sequence** | **T_{1/2} [min]** |
|---|---|---|---|---|
| **8** | *N*PEG₄-(IETDV)₂ | L-TAT | YGRKKRRQRRR- | 543 ± 60 |
| **9** | *N*PEG₄-(IETDV)₂ | L-TAT | Ac-YGRKKRRQRRR- | 229 ± 129 |
| **38** | *N*PEG₄-(IETDV)₂ | D-TAT | Ac-ygrkkrrqrrr- | 1440 ± 0 |
| **40** | *N*PEG₄-(IETDV)₂ | mTAT | Ac-rRrGrKkRr- | 1440 ± 0 |
| **42** | *N*PEG₄-(IETDV)₂ | riTAT | Ac-rrrqrrkkr- | 1440 ± 0 |
| **35** | *N*PEG₄-(IETDV)₂ | L-Arg₉ | Ac-RRRRRRRRR- | 1492 ± 114 |
| **44** | *N*PEG₄-(IETDV)₂ | D-Arg₉ | Ac-rrrrrrrrr- | 1440 ± 0 |
| **46** | *N*PEG₄-(IETDV)₂ | L-Pen | Ac- RQIKIWFQNRRMKWKK- | 94 ± 8 |
| **48** | *N*PEG₄-(IETDV)₂ | D-Pen | Ac-rqikiwfqnrrmkwkk- | 1440 ± 0 |
| **50** | *N*PEG₄-(IETDV)₂ | L-DPV3 | Ac- RKKRRRESRKKRRRES- | 169 ± 20 |
| **52** | *N*PEG₄-(IETDV)₂ | L-pVEC | Ac- LLIILRRRIRKQAHAHSK- | 49 ± 9 |
| **54** | *N*PEG₄-(IETDV)₂ | L-MAP | Ac- KLALKLALKALKAALKLA- | 13 ± 4 |
| **56** | *N*PEG₄-(IETDV)₂ | L-TP2 | Ac-PLlYLRLLRGQF- | 910 ± 0 |
| **58** | *N*PEG₄-(IETDV)₂ | D-TP2 | Ac-pliylrllrGqf- | 1440 ± 0 |
| **60** | *N*PEG₄-(IETDV)₂ | MiniAp4 | Ac-\|(Dap)KAPETALD\|- | 1440 ± 0 |
| **62** | *N*PEG₄-(IETDV)₂ | CPP12 | \|Ff(NaI2)RrRrQ\|GABA-K- | 1440 ± 0 |

| | | | | |
|---|---|---|---|---|
| Data represented as n ≥ 3; Ac = *N*-terminal acetylation. | | | | |

### Example 4: Determination of membrane permeability and cellular uptake of ligands

The membrane permeability of compounds **10** to **75** was determined in HeLa cells stably expressing HaloGFP exclusively located in the cytosol. Cells were seeded at a density of 40.000 cells/well one day prior to the experiment. After the growth media was aspirated and replaced by 100 µL of Opti-MEM, 25 µL of a prepared serial dilution of the ligand in Opti-MEM was added to the cells (constant DMSO concentration), and the plate was incubated for 4 h at 37 °C and 5% CO₂. The contents of the wells were aspirated, and cells were washed with fresh Opti-MEM for 15 min. After aspiration of the wash, the cells were incubated with TAMRA-CA (5 µM) for 15 min. After aspiration of the chase solution, cells were washed with Opti-MEM for 30 min. Following removal of the wash, cells were trypsinized, resuspended in PBS (2% FBS), and analyzed using a benchtop flow cytometer. Using no ligand and no TAMRA-CA control well, the obtained fluorescence intensity data was normalized and plotted as dose-response curves. Reported CP₅₀ values represent half-maximum red fluorescence which behaves inverse to cell penetration of the ligand.

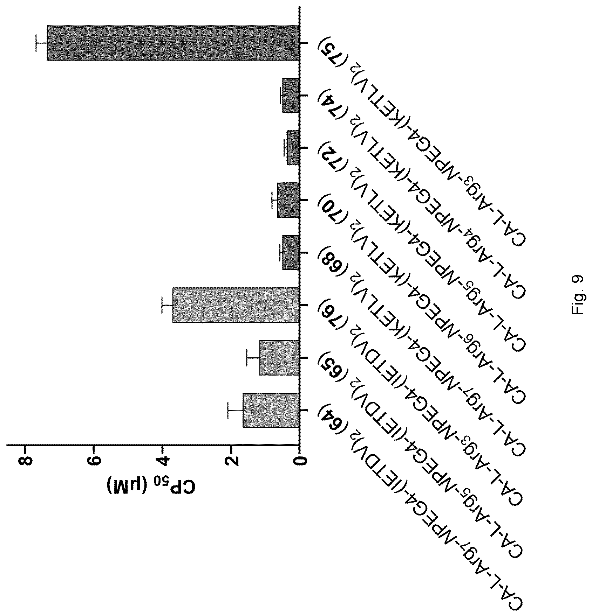

This example describes how to determine the membrane permeability and cellular uptake of CA-tagged compounds binding to PDZ1-2 of PSD-95. The results are presented in tables 10 to 12, and Figures 7 to 9. In conclusion, the tested compounds show significantly different cellular uptake efficiencies, which greatly depend on the employed CPP. Surprisingly, the CPP-tag L-Arg₉ (SEQ ID NO 12) and L-pVEC (SEQ ID NO: 18) result in the lowest CP₅₀ values (Fig. 8). Dependent on the dimeric ligand, L-Arg₉ further promotes highly efficient cellular uptake with CP₅₀ values below 1 µM (Fig. 7) suggesting superior BBB penetration and intracellular delivery. In addition, the L-Arg₉ CPP-tag can be truncated to 7, 6, 5, or 4 Arginines (see table 12) without losing its cell-permeable properties (Fig. 9).

**Table 10. Membrane permeability and cellular uptake, CP₅₀ presented as mean ± SEM, for NPEG₄ dimeric ligands fused to L-TAT and L-Arg₉ (Figure 7).**

| **Compound** | **Ligand** | **CPP** | **CPP sequence** | **CP₅₀ [µM]** |
|---|---|---|---|---|
| **10** | *N*PEG₄-(IETDV)₂ | L-TAT | CA-YGRKKRRQRRR- | 55.1 ± 3,0 |
| **12** | *N*PEG₄-(KETLV)₂ | L-TAT | CA-YGRKKRRQRRR- | 72.5 ± 4,4 |
| **14** | *N*PEG₄-(KETTV)₂ | L-TAT | CA-YGRKKRRQRRR- | 78.4 ± 3,9 |
| **16** | *N*PEG₄-(KETVV)₂ | L-TAT | CA-YGRKKRRQRRR- | 81.8 ± 1,2 |
| **18** | *N*PEG₄-(KETRV)₂ | L-TAT | CA-YGRKKRRQRRR- | 47.7 ± 1,0 |
| **20** | *N*PEG₄-(ISTDV)₂ | L-TAT | CA-YGRKKRRQRRR- | 41.9 ± 1,6 |
| **22** | *N*PEG₄-(VETVV)₂ | L-TAT | CA-YGRKKRRQRRR- | 86.1 ± 4,5 |
| **36** | *N*PEG₄-(IETDV)₂ | L-Arg₉ | CA-RRRRRRRRR- | 3.25 ± 0,19 |
| **24** | *N*PEG₄-(KETLV)₂ | L-Arg₉ | CA-RRRRRRRRR- | 0.78 ± 0,05 |
| **26** | *N*PEG₄-(KETTV)₂ | L-Arg₉ | CA-RRRRRRRRR- | 0.74 ± 0,09 |
| **28** | *N*PEG₄-(KETVV)₂ | L-Arg₉ | CA-RRRRRRRRR- | 0.79 ± 0,06 |
| **30** | *N*PEG₄-(KETRV)₂ | L-Arg₉ | CA-RRRRRRRRR- | 0.68 ± 0,05 |
| **32** | *N*PEG₄-(ISTDV)₂ | L-Arg₉ | CA-RRRRRRRRR- | 1.05 ± 0,15 |
| **34** | *N*PEG₄-(VETVV)₂ | L-Arg₉ | CA-RRRRRRRRR- | 0.84 ± 0,15 |

| | | | | |
|---|---|---|---|---|
| Data represented as n ≥ 3; Ac = *N*-terminal acetylation. | | | | |

**Table 11. Membrane permeability and cellular uptake, CP₅₀ presented as mean ± SEM, for NPEG₄-(IETDV)₂ dimeric ligands fused to various CPP-tags (Figure 8).**

| **Compound** | **Ligand** | **CPP** | **CPP sequence** | **CP₅₀ [µM]** |
|---|---|---|---|---|
| **10** | *N*PEG₄-(IETDV)₂ | L-TAT | CA-YGRKKRRQRRR- | 55.1 ± 3,0 |
| **39** | *N*PEG₄-(IETDV)₂ | D-TAT | CA-yGrkkrrqrrr- | 10.7 ± 0,7 |
| **41** | *N*PEG₄-(IETDV)₂ | mTAT | CA-rRrGrKkRr- | 180 ± 8,2 |
| **43** | *N*PEG₄-(IETDV)₂ | riTAT | CA-rrrqrrkkr- | 221 ± 36 |
| **36** | *N*PEG₄-(IETDV)₂ | L-Arg₉ | CA-RRRRRRRRR- | 3.3 ± 0,2 |
| **45** | *N*PEG₄-(IETDV)₂ | D-Arg₉ | CA-rrrrrrrrr- | 161 ± 25 |
| **47** | *N*PEG₄-(IETDV)₂ | L-Pen | CA- RQIKIWFQNRRMKWKK- | 5.0 ± 0,4 |
| **49** | *N*PEG₄-(IETDV)₂ | D-Pen | CA-rqikiwfqnrrmkwkk- | 18.8 ± 1,5 |
| **51** | *N*PEG₄-(IETDV)₂ | L-DPV3 | CA- RKKRRRESRKKRRRES- | 3.2 ± 0,5 |
| **53** | *N*PEG₄-(IETDV)₂ | L-pVEC | CA- LLIILRRRIRKQAHAHSK- | 1.7 ± 0,1 |
| **55** | *N*PEG₄-(IETDV)₂ | L-MAP | CA- KLALKLALKALKAALKLA- | 3.7 ± 0,3 |
| **57** | *N*PEG₄-(IETDV)₂ | L-TP2 | CA-PLIYLRLLRGQF- | 17.9 ± 0,3 |
| **59** | *N*PEG₄-(IETDV)₂ | D-TP2 | CA-pliylrllrGqf- | 67.2 ± 5,3 |
| **61** | *N*PEG₄-(IETDV)₂ | MiniAp4 | CA-\|(Dap)KAPETALD\|- | 13.2 ± 0,4 |
| **63** | *N*PEG₄-(IETDV)₂ | CPP12 | ;Ff(Nal2)RrRrQ;GABA- K(CA)- | 11.2 ± 0,7 |

| | | | | |
|---|---|---|---|---|
| Data represented as n ≥ 3; Ac = *N*-terminal acetylation. | | | | |

**Table 12. Membrane permeability and cellular uptake, CP₅₀ presented as mean ± SEM, for NPEG₄-(IETDV)₂ and NPEG₄-(KETLV)₂ dimeric ligands fused to polyArg CPP-tags (Figure 9).**

| **Compound** | **Ligand** | **CPP** | **CPP sequence** | **CP₅₀ [µM]** |
|---|---|---|---|---|
| **64** | *N*PEG₄-(IETDV)₂ | L-Arg₇ | CA-RRRRRRR- | 1.66 ± 0.43 |
| **65** | *N*PEG₄-(IETDV)₂ | L-Arg₅ | CA-RRRRR- | 1.17 ± 0.37 |
| **66** | *N*PEG₄-(IETDV)₂ | L-Arg₃ | CA-RRR- | 3.71 ± 0.30 |
| **68** | *N*PEG₄-(KETLV)₂ | L-Arg₇ | CA-RRRRRRR- | 0.58 ± 0.09 |
| **70** | *N*PEG₄-(KETLV)₂ | L-Arg₉ | CA-RRRRRR- | 0.51 ± 0.08 |
| **72** | *N*PEG₄-(KETLV)₂ | L-Arg₅ | CA-RRRRR- | 0.66 ± 0.14 |
| **74** | *N*PEG₄-(KETLV)₂ | L-Arg₄ | CA-RRRR- | 0.37 ± 0.08 |
| **75** | *N*PEG₄-(KETLV)₂ | L-Arg₃ | CA-RRR- | 7.36 ± 0.31 |

| | | | | |
|---|---|---|---|---|
| Data represented as n ≥ 3; Ac = *N*-terminal acetylation. | | | | |

### Example 5: Determination of in vitro plasma stability

The *in vitro* plasma stability of the compounds **8, 23, 35, 67, 69, 71** and **73 was** determined by incubating 100 µM of ligand in undiluted pooled human plasma at 37 °C for 1440 minutes. At selected time points during the incubation, the ligands were extracted from the assay matrix by adding assay matrix to 6M Urea followed by precipitation using 20% TCA in acetone. After overnight incubation, the samples were filtered and analysed by UPLC to determine the amount of ligand remaining. LC-MS analysis was performed to confirm ligand integrity and identify cleavage sites.

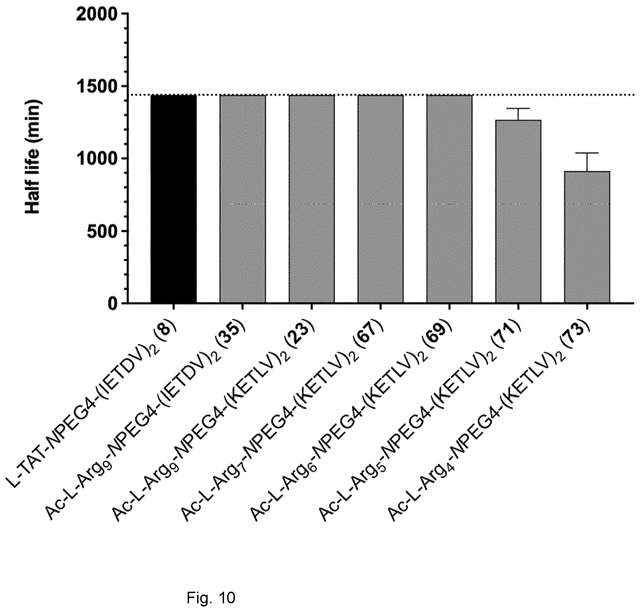

This example describes how to determine the *in vitro* plasma stability of ligands binding to PDZ1-2 of PSD-95. The results are presented in table 13, and Figure 10. In conclusion, the L-Arg₉ CPP-tag can be truncated to 7, 6, 5, or 4 Arginines (see table 13) without being significantly less stable to human plasma.

**Table 13. In vitro plasma stability, half-life times presented as mean ± SEM, for NPEG₄-(KETLV)₂ dimeric ligands fused to polyArg CPP-tags (Figure 10).**

| **Compound** | **Ligand** | **CPP** | **CPP sequence** | **T_{1/2} [min]** |
|---|---|---|---|---|
| **8** | *N*PEG₄-(IETDV)₂ | L-TAT | YGRKKRRQRRR- | 1440 ± 0 |
| **35** | *N*PEG₄-(IETDV)₂ | L-Arg₉ | Ac-RRRRRRRRR- | 1440 ± 0 |
| **23** | *N*PEG₄-(KETLV)₂ | L-Arg₉ | Ac-RRRRRRRRR- | 1440 ± 0 |
| **67** | *N*PEG₄-(KETLV)₂ | L-Arg₇ | Ac-RRRRRRR- | 1440 ± 0 |
| **69** | *N*PEG₄-(KETLV)₂ | L-Arg₆ | Ac-RRRRRR- | 1440 ± 0 |
| **71** | *N*PEG₄-(KETLV)₂ | L-Arg₅ | Ac-RRRRR- | 1266 ± 138 |
| **73** | *N*PEG₄-(KETLV)₂ | L-Arg₄ | Ac-RRRR- | 912 ± 216 |

| | | | | |
|---|---|---|---|---|
| Data represented as n ≥ 3; Ac = N-terminal acetylation. | | | | |

### Example 6: Determination of in vivo brain levels post i.v. injection

Female Balb/c mice (4 groups of 3 animals) are dosed with 18 nmol/g bodyweight with compounds of interested using isotonic saline as vehicle. Group 1 is used to obtain blood concentration profiles post i.v. injection at selected time points (max. 4h post injection). Groups 2 to 4 are used to determine blood and brain concentrations at three different timepoints. Animals are therefore terminated at selected timepoints post i.v. injection, relevant tissue is collected and analysed using appropriate methods (LC-MS/MS).

The example describes how to determine *in vivo* brain levels of ligands binding to PDZ1-2 of PSD-95. Furthermore, blood to brain ratios and relative tissue targeting efficiencies are determined, informing about a compounds ability to cross the blood-brain barrier and reach the target in the brain.

### Example 7: Determination of in vivo neuroprotective effect against stroke induced damage using a pMCAO mouse model.

For experimental and surgical details see Bach et al, Proc. Natl. Acad. Sci. USA, 2012, 109, 3317. In brief, the extent of ischemic infarct is being measured in a randomized, double-blinded, placebo-controlled study using a permanent middle cerebral artery occlusion (pMCAO) model in C57BL/6J mice. Following anaesthesia and artery occlusion, animals are dosed 30 min post-surgery. Animals surviving 6h post-surgery were terminated, and the brain carefully removed. Following fixation and slicing of the brains, infarct volumetric analysis was performed, and the total infarct volume calculated. The example describes how to determine the *in vivo* neuroprotective effect of ligans binding PDZ1-2 of PSD-95. Read out parameter is the infarct volume in the compound treated group versus placebo controlled group.

### Sequences

| **SEQ ID NO** | **Sequence** | **Comment** |
|---|---|---|
| 1 | IETDV | |
| 2 | KETLV | |
| 3 | KETTV | |
| 4 | KETVV | |
| 5 | KETRV | |
| 6 | ISTDV | |
| 7 | VETVV | |
| 8 | YGRKKRRQRRR | L-TAT |
| 9 | yGrkkrrqrrr | D-TAT |
| 10 | rRrGrKkRr | mTAT |
| 11 | rrrqrrkkr | riTAT |
| 12 | RRRRRRRRR | L-Arg₉ |
| 13 | rrrrrrrrr | D-Arg₉ |
| 14 | RQIKIWFQNRRMKWKK | L-Pen |
| 15 | rqikiwfqnrrmkwkk | D-Pen |
| 16 | KLALKLALKALKAALKLA | L-MAP |
| 17 | RKKRRRESRKKRRRES | L-DPV3 |
| 18 | LLIILRRRIRKQAHAHSK | L-pVEC |
| 19 | PLIYLRLLRGQF | L-TP2 |
| 20 | pliylrllrGqf | D-TP2 |
| 21 | \|(Dap)KAPETALD\| | MiniAp4 |
| | | Side chain-to-side chain macrolactamization through D and Dap Dap is L-2,3-diaminopropionic acid |
| 22 | \|Ff(Nal2)RrRrQ\|GABA-K | CPP12 |
| | | Head-to-tail macrolactamization through |
| | | Q and F |
| | | Nal2 is L-2-naphthylalanine |
| 23 | Ac-YGRKKRRQRRR- | L-TAT |
| | | N-terminal acetylated |
| 24 | CA-YGRKKRRQRRR- | L-TAT |
| | | N-terminal conjugated to chloroalkane (CA) |
| 25 | Ac-RRRRRRRRR- | L-Arg₉ |
| | | N-terminal acetylated |
| 26 | CA-RRRRRRRRR- | L-Arg₉ |
| | | N-terminal conjugated to chloroalkane (CA) |
| 27 | Ac-ygrkkrrqrrr- | D-TAT |
| | | N-terminal acetylated |
| 28 | CA-ygrkkrrqrrr- | D-TAT |
| | | N-terminal conjugated to chloroalkane (CA) |
| 29 | Ac-rRrGrKkRr- | mTAT |
| | | N-terminal acetylated |
| 30 | CA-rRrGrKkRr- | mTAT |
| | | N-terminal conjugated to chloroalkane (CA) |
| 31 | Ac-rrrqrrkkr- | riTAT |
| | | N-terminal acetylated |
| 32 | CA-rrrqrrkkr- | riTAT |
| | | N-terminal conjugated to chloroalkane (CA) |
| 33 | Ac-rrrrrrrrr- | D-Arg₉ |
| | | N-terminal acetylated |
| 34 | CA-rrrrrrrrr- | D-Arg₉ |
| | | N-terminal conjugated to chloroalkane (CA) |
| 35 | Ac- RQIKIWFQNRRMKWKK- | L-Pen |
| | | N-terminal acetylated |
| 36 | CA- RQIKIWFQNRRMKWKK- | L-Pen |
| | | N-terminal conjugated to chloroalkane (CA) |
| 37 | Ac-rqikiwfqnrrmkwkk- | D-Pen |
| | | N-terminal acetylated |
| 38 | CA-rqikiwfqnrrmkwkk- | D-Pen |
| | | N-terminal conjugated to chloroalkane (CA) |
| 39 | Ac- RKKRRRESRKKRRRES- | L-DPV3 |
| | | N-terminal acetylated |
| 40 | CA- RKKRRRESRKKRRRES- | L-DPV3 |
| | | N-terminal conjugated to chloroalkane (CA) |
| 41 | Ac- LLIILRRRIRKQAHAHSK- | L-pVEC |
| | | N-terminal acetylated |
| 42 | CA- LLIILRRRIRKQAHAHSK- | L-pVEC |
| | | N-terminal conjugated to chloroalkane (CA) |
| 43 | Ac- KLALKLALKALKAALKLA- | L-MAP |
| | | N-terminal acetylated |
| 44 | CA- KLALKLALKALKAALKLA- | L-MAP |
| | | N-terminal conjugated to chloroalkane (CA) |
| 45 | Ac-PLIYLRLLRGQF- | L-TP2 |
| | | N-terminal acetylated |
| 46 | CA-PLIYLRLLRGQF- | L-TP2 |
| | | N-terminal conjugated to chloroalkane (CA) |
| 47 | Ac-pliylrllrGqf- | D-TP2 |
| | N-terminal acetylated | N-terminal acetylated |
| 48 | CA-pliylrllrGqf- | D-TP2 |
| | | N-terminal conjugated to chloroalkane (CA) |
| 49 | Ac-\|(Dap)KAPETALD\|- | MiniAp4 |
| | N-terminal acetylated | N-terminal acetylated |
| 50 | CA-\|(Dap)KAPETALD\|- | MiniAp4 |
| | | N-terminal conjugated to chloroalkane |
| 51 | \|Ff(Nal2)RrRrQ\|GABA-K- | CPP12 |
| | | No modification of the side chain of K |
| 52 | ;Ff(Nal2)RrRrQ;GABA- K(CA)- | CPP12 |
| | | Chloroalkane (CA) is conjugated to the amine in the side chain of K |
| 53 | X₂TX₃V | X₂ is E or S |
| | | X₃ is L, T, V, R or D |
| 54 | X₁X₂TX₃V | X₁ is K, V or I |
| | | X₂ is E or S |
| | | X₃ is L, T, V, R or D |
| 55 | X₅TX₆V | X₅ is E or S |
| | | X₆ is L, T, V, R or D |
| 56 | X₄X₅TX₆V | X₄ is K, V or I |
| | | X₅ is E or S |
| | | X₆ is L, T, V, R or D |
| 57 | RRRRRRRR | L-Arg₈ |
| 58 | RRRRRRR | L-Arg₇ |
| 59 | RRRRRR | L-Arg₆ |
| 60 | RRRRR | L-Arg₅ |
| 61 | RRRR | L-Arg₄ |
| 62 | Ac-RRRRRRRR- | L-Arg₈ |
| | | N-terminal acetylated |
| 63 | Ac-RRRRRRR- | L-Arg₇ |
| | | N-terminal acetylated |
| 64 | Ac-RRRRRR- | L-Arg₆ |
| | | N-terminal acetylated |
| 65 | Ac-RRRRR- | L-Arg₅ |
| | | N-terminal acetylated |
| 66 | Ac-RRRR- | L-Arg₄ |
| | | N-terminal acetylated |
| 67 | CA-RRRRRRRR- | L-Arg₈ |
| | | N-terminal conjugated to chloroalkane (CA) |
| 68 | CA-RRRRRRR- | L-Arg₇ |
| | | N-terminal conjugated to chloroalkane (CA) |
| 69 | CA-RRRRRR- | L-Arg₆ |
| | | N-terminal conjugated to chloroalkane (CA) |
| 70 | CA-RRRRR- | L-Arg₅ |
| | | N-terminal conjugated to chloroalkane (CA) |
| 71 | CA-RRRR- | L-Arg₄ |
| | | N-terminal conjugated to chloroalkane (CA) |

### References

Aarts, M., Liu, Y., Liu, L., Besshoh, S., Arundine, M., Gurd, J.W., Wang, Y.T., Salter, M.W., Tymianski, M., 2002. Treatment of ischemic brain damage by perturbing NMDA receptor-PSD-95 protein interactions. Science 298, 846-850.
J. T. Andreasen, A. Bach, M. Gynther, A. Nasser, J. Mogensen, K. Strømgaard, D. S. Pickering, UCCB01-125, a dimeric inhibitor of PSD-95, reduces inflammatory pain without disrupting cognitive or motor performance: comparison with the NMDA receptor antagonist MK-801, Neuropharmacology, 67, 193-200 (2013).
Bach, A., Chi, C.N., Pang, G.F., Olsen, L., Kristensen, A.S., Jemth, P., Strømgaard, K., Design and synthesis of highly potent and plasma-stable dimeric inhibitors of the PSD-95-NMDA receptor interaction. Angew. Chem. Int. Ed. 48, 9685-9689 (2009).
Bach, A., Clausen, B.H., Møller, M., Vestergaard, B., Chi, C.N., Round, A., Sørensen, P.L., Nissen, K.B., Kastrup, J.S., Gajhede, M., Jemth, P., Kristensen, A.S., Lundström, P., Lambertsen, K.L., Strømgaard, K., A high-affinity, dimeric inhibitor of PSD-95 bivalently interacts with PDZ1-2 and protects against ischemic brain damage. Proc. Natl. Acad. Sci. U.S.A. 109, 3317-3322 (2012).
Dawson, V.L., Dawson, T.M., London, E.D., Bredt, D.S., Snyder, S.H., 1991. Nitric oxide mediates glutamate neurotoxicity in primary cortical cultures. Proc. Natl. Acad. Sci. U. S. A 88, 6368-6371.
Deprey, K.; Kritzer, J. A., 2020. Quantitative measurement of cytosolic penetration using the chloroalkane penetration assay. Method. Enzymol. 641, 277-309.
Hill et al. 2020. Efficacy and safety of nerinetide for the treatment of acute ischaemic stroke (ESCAPE-NA1): A multicentre, double-blind, randomised controlled trial. Lancet 395, 878-887.
Huang, Z., Huang, P.L., Panahian, N., Dalkara, T., Fishman, M.C., Moskowitz, M.A., 1994. Effects of cerebral ischemia in mice deficient in neuronal nitric oxide synthase. Science 265, 1883-1885.
Kornau, H. C., Schenker, L. T., Kennedy, M. B., Seeburg, P. H., 1995. Domain interaction between NMDA receptor subunits and the postsynaptic density protein PSD-95. Science 269, 1737-1740.
Kucharz, K., Rasmussen, I.S., Bach, A., Strømgaard, K., Lauritzen, M., PSD-95 uncoupling from NMDA receptors by Tat-N-dimer ameliorates neuronal depolarization in cortical spreading depression. J. Cereb. Blood Flow Metab. 37, 1820-1828 (2017).
Mayor-Nunez, D., Ji, Z., Sun, X., Teves, L., Garman, J. D., Tymianski, M., 2021. Plasmin-resistant PSD-95 inhibitors resolve effect-modifying drug-drug interactions between alteplase and nerinetide in acute stroke, Sci. Transl. Med. 13, eabb1498.
Peraro, L.; Deprey, K. L.; Moser, M. K.; Zou, Z.; Ball, H. L.; Levine, B.; Kritzer, J. A., 2018. Cell Penetration Profiling Using the Chloroalkane Penetration Assay. J. Am. Chem. Soc. 140, 11360-11369.
Sattler, R., Xiong, Z., Lu, W.Y., Hafner, M., MacDonald, J.F., Tymianski, M., 1999. Specific coupling of NMDA receptor activation to nitric oxide neurotoxicity by PSD-95 protein. Science 284, 1845-1848.

## Claims

1. A PSD-95 inhibitor comprising:
i. a first peptide (P₁) comprising or consisting of the amino acid sequence X₂TX₃V (SEQ ID NO: 53), wherein
X₂ is selected from the group consisting of E and S;
X₃ is selected from the group consisting of L, T, V, R and D;
ii. a second peptide (P₂) comprising or consisting of the amino acid sequence X₅TX₆V (SEQ ID NO: 55), wherein
X₅ is selected from the group consisting of E and S;
X₆ is selected from the group consisting of L, T, V, R and D; and
iii. a Cell Penetrating Peptide (CPP) selected from the group consisting of:
a) a poly-L-arginine peptide (poly-Arg) consisting of 3 to 9 L-arginine residues;
b) yGrkkrrqrrr (SEQ ID NO: 9, D-TAT);
c) RQIKIWFQNRRMKWKK (SEQ ID NO: 14, L-Pen);
d) rqikiwfqnrrmkwkk (SEQ ID NO: 15, D-Pen);
e) RKKRRRESRKKRRRES (SEQ ID NO: 17, L-DPV3);
f) LLIILRRRIRKQAHAHSK (SEQ ID NO: 18, L-pVEC);
g) KLALKLALKALKAALKLA (SEQ ID NO: 16, L-MAP);
h) PLIYLRLLRGQF (SEQ ID NO: 19, L-TP2);
i) |(Dap)KAPETALD| (SEQ ID NO: 21, MiniAp4); and
j) |Ff(Nal2)RrRrQ|GABA-K (SEQ ID NO: 22, CPP12),
wherein the CPP is linked to a linker via its C-terminal, and P₁ and P₂ are conjugated to the linker via their N-termini, and the PSD-95 inhibitor has the general structure of Formula (I): 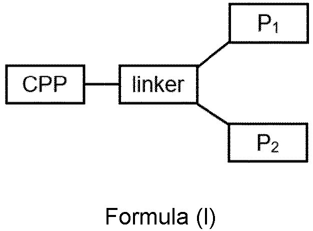
or a pharmaceutically acceptable salt thereof.

2. The PSD-95 inhibitor according to claim 1, wherein P₁ comprises or consists of the sequence X₁X₂TX₃V (SEQ ID NO: 54), wherein
X₁ is selected from the group consisting of K, V and I;
X₂ is selected from the group consisting of E and S; and
X₃ is selected from the group consisting of L, T, V, R and D;
and wherein P₂ comprises or consists of the sequence X₄X₅TX₆V (SEQ ID NO: 56), wherein
X₄ is selected from the group consisting of K, V and I;
X₅ is selected from the group consisting of E and S; and
X₆ is selected from the group consisting of L, T, V, R and D.

3. The PSD-95 inhibitor according to any one of the preceding claims, wherein P₁ and/or P₂ consist of 4 to 10 amino acid residues, such as 5 amino acid residues.

4. The PSD-95 inhibitor according to any one of the preceding claims, wherein X₃ and X₆ are selected from the group consisting of L, T, V, and R.

5. The PSD-95 inhibitor according to any one of the preceding claims, wherein the linker comprises one or more PEG units wherein at least one oxygen atom of one of the PEG units is replaced with a nitrogen atom to give NPEG, and wherein the CPP is linked to the nitrogen atom of the linker by an amide bond, and the PSD-95 inhibitor has the general structure of Formula (III): 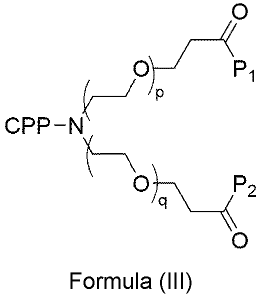 wherein
p is an integer 0 to 10; and
q is an integer 0 to 10.

6. The PSD-95 inhibitor according to any one of the preceding claims, wherein P₁ and P₂ consist of the amino acid sequence KETLV (SEQ ID NO: 2), KETTV (SEQ ID NO: 3), KETVV (SEQ ID NO: 4), KETRV (SEQ ID NO: 5), ISTDV (SEQ ID NO: 6), or VETVV (SEQ ID NO: 7).

7. The PSD-95 inhibitor according to any one of the preceding claims, wherein the PSD-95 inhibitor has the general structure of Formula (VI): 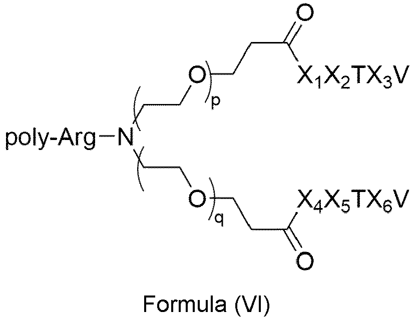 wherein
X₁ is selected from the group consisting of K, V and I;
X₂ is selected from the group consisting of E and S; and
X₃ is selected from the group consisting of L, T, V, R and D.
X₄ is selected from the group consisting of K, V and I;
X₅ is selected from the group consisting of E and S;
X₆ is selected from the group consisting of L, T, V, R and D.
p is an integer 0 to 10;
q is an integer 0 to 10; and
poly-Arg is a poly-L-arginine peptide consisting of 3 to 9 L-arginine residues.

8. The PSD-95 inhibitor according to any one of the preceding claims, wherein the CPP is RRRRRRRRR (SEQ ID NO: 12), RRRRRRRR (SEQ ID NO: 57), RRRRRRR (SEQ ID NO: 58), RRRRRR (SEQ ID NO: 59), RRRRR (SEQ ID NO: 60), RRRR (SEQ ID NO: 61), or RRR.

9. The PSD-95 inhibitor according to any one of the preceding claims, wherein the N-terminal of the CPP is acetylated or wherein the N-terminal of the CPP is conjugated to a chloroalkane tag (CA), which has the structure of:

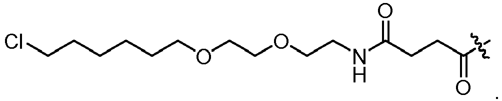

10. The PSD-95 inhibitor according to claim 1, wherein the PSD-95 inhibitor has the general structure of Formula (XXVIII): 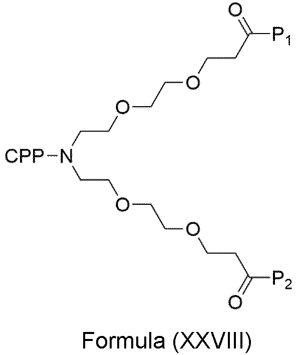
wherein P₁, P₂ and CPP are:
| | **P₁** | **P₂** | **CPP** | |
|---|---|---|---|---|
| i. | KETLV (SEQ ID NO: 2) | KETLV (SEQ ID NO: 2) | L-Arg₉ | RRRRRRRRR (SEQ ID NO: 12) |
| ii. | KETLV (SEQ ID NO: 2) | KETLV (SEQ ID NO: 2) | L-Arg₇ | RRRRRRR (SEQ ID NO: 58) |
| iii. | KETLV (SEQ ID NO: 2) | KETLV (SEQ ID NO: 2) | L-Arg₆ | RRRRRR (SEQ ID NO: 59) |
| iv. | KETLV (SEQ ID NO: 2) | KETLV (SEQ ID NO: 2) | L-Arg₅ | RRRRR (SEQ ID NO: 60) |
| v. | KETLV (SEQ ID NO: 2) | KETLV (SEQ ID NO: 2) | L-Arg₄ | RRRR (SEQ ID NO: 61) |
| vi. | KETLV (SEQ ID NO: 2) | KETLV (SEQ ID NO: 2) | L-Arg₃ | RRR |
| vii. | KETTV (SEQ ID NO: 3) | KETTV (SEQ ID NO: 3) | L-Arg₉ | RRRRRRRRR (SEQ ID NO: 12) |
| viii. | KETVV (SEQ ID NO: 4) | KETVV (SEQ ID NO: 4) | L-Arg₉ | RRRRRRRRR (SEQ ID NO: 12) |
| ix. | KETRV (SEQ ID NO: 5) | KETRV (SEQ ID NO: 5) | L-Arg₉ | RRRRRRRRR (SEQ ID NO: 12) |
| x. | ISTDV (SEQ ID NO: 6) | ISTDV (SEQ ID NO: 6) | L-Arg₉ | RRRRRRRRR (SEQ ID NO: 12) |
| xi. | VETVV (SEQ ID NO: 7) | VETVV (SEQ ID NO: 7) | L-Arg₉ | RRRRRRRRR (SEQ ID NO: 12) |
| xii. | IETDV (SEQ ID NO: 1) | IETDV (SEQ ID NO: 1) | L-Arg₉ | RRRRRRRRR (SEQ ID NO: 12) |
| xiii. | IETDV (SEQ ID NO: 1) | IETDV (SEQ ID NO: 1) | L-Arg₇ | RRRRRRR (SEQ ID NO: 58) |
| xiv. | IETDV (SEQ ID NO: 1) | IETDV (SEQ ID NO: 1) | L-Arg₅ | RRRRR (SEQ ID NO: 60) |
| xv. | IETDV (SEQ ID NO: 1) | IETDV (SEQ ID NO: 1) | L-Arg₃ | RRR |
and wherein the N-terminal of the CPP is optionally acetylated,
or
wherein the PSD-95 inhibitor has the general structure of Formula (XXVIII),
wherein P₁ and P₂ consist of the amino acid sequence IETDV (SEQ ID NO: 1) and the CPP is selected from the group consisting of rqikiwfqnrrmkwkk (SEQ ID NO: 15, D-Pen); LLIILRRRIRKQAHAHSK (SEQ ID NO: 18, L-pVEC);
PLIYLRLLRGQF (SEQ ID NO: 19, L-TP2); |(Dap)KAPETALD| (SEQ ID NO: 21, MiniAp4); and |Ff(Nal2)RrRrQ|GABA-K (SEQ ID NO: 22, CPP12).

11. The PSD-95 inhibitor according to claim 1, wherein the PSD-95 inhibitor is selected from the group consisting of formulas (X) to (XXIII):

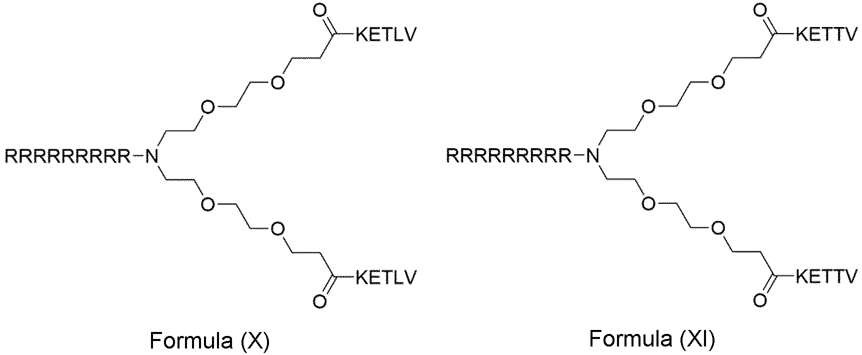

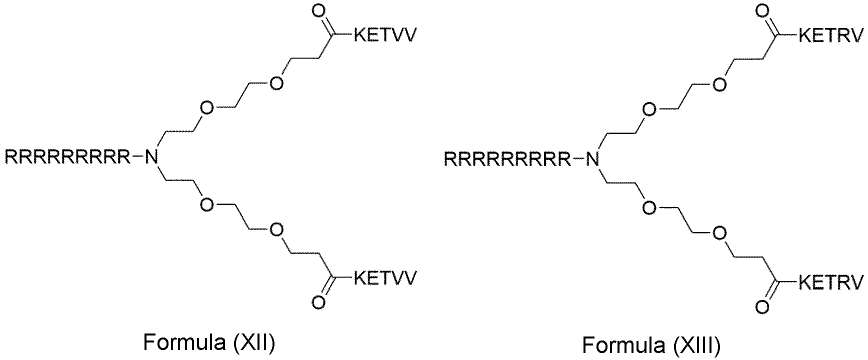

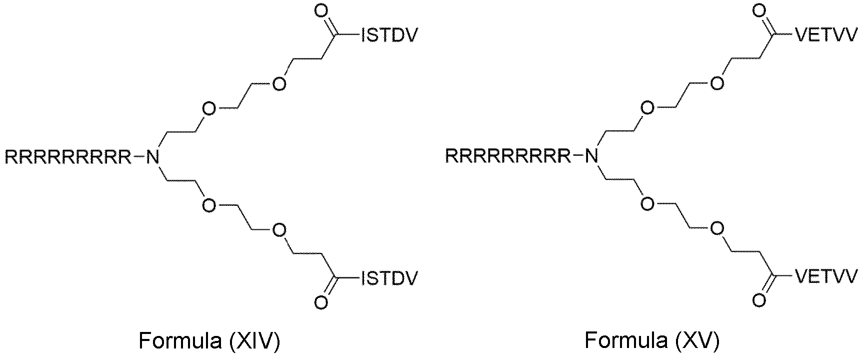

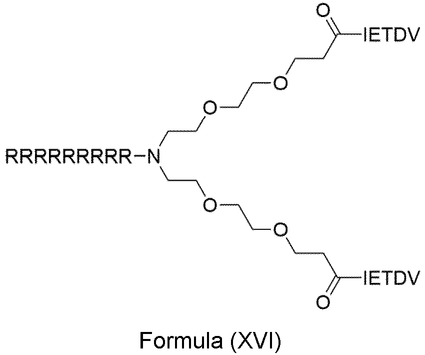

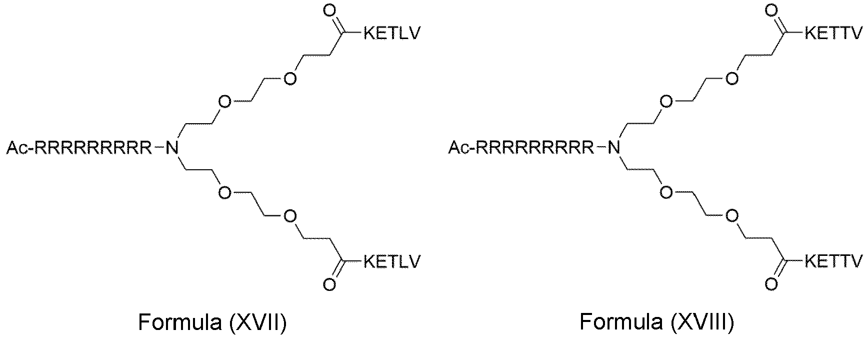

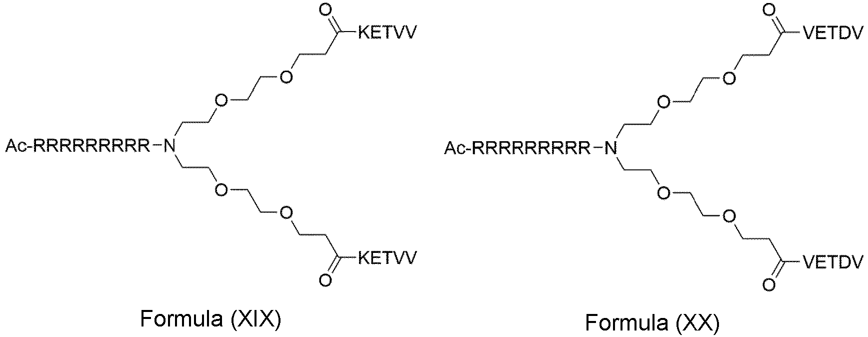

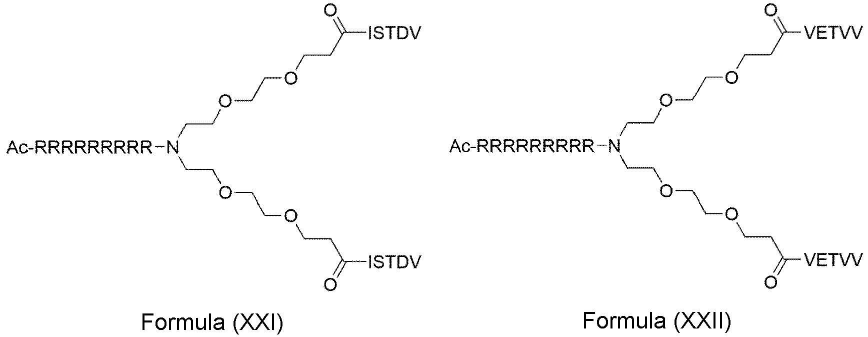

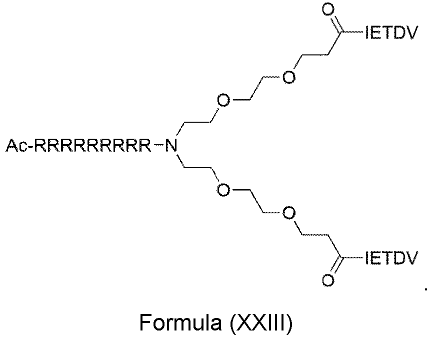

12. The PSD-95 inhibitor according to any one of claims 1 to 3, 5, 9 or 10, wherein P₁ and P₂ consist of the amino acid sequence IETDV (SEQ ID NO: 1) and the CPP is selected from the group consisting of rqikiwfqnrrmkwkk (SEQ ID NO: 15, D-Pen); LLIILRRRIRKQAHAHSK (SEQ ID NO: 18, L-pVEC); PLIYLRLLRGQF (SEQ ID NO: 19, L-TP2); |(Dap)KAPETALD| (SEQ ID NO: 21, MiniAp4); and |Ff(Nal2)RrRrQ|GABA-K (SEQ ID NO: 22, CPP12).

13. A PSD-95 inhibitor according to any one of the preceding claims for use as a medicament.

14. A PSD-95 inhibitor according to any one claims 1 to 12 for use in treating, preventing, reducing and/or delaying development of an excitotoxic-related disease such as stroke, for example selected from acute ischemic stroke and subarachnoid hemorrhage.

15. A method for manufacturing the PSD-95 inhibitor according to any one of claims 1 to 12 comprising the general steps of:
i.
a) Synthesizing a peptide P₁/P₂;
b) Dimerizing the peptide of a) with an OPEGₙ or NPEGₙ linker, wherein 'n' is the number of PEG moieties; and
c) Attaching a CPP tag at the N of the NPEG linker, for example by using an automated peptide synthesizer;
or
ii.
a) providing a Ns-NPEG diacid linker;
b) preparing a peptide P₁/P₂ using Fmoc-based solid-phase peptide synthesis;
c) dimerizing Fmoc-deprotected peptide P₁/P₂ with the Ns-NPEG diacid linker forming a linker-dimer conjugate; and
d) attaching a CPP to the N of the NPEG linker of the linker-dimer conjugate, for example by using an automated peptide synthesizer.

## Patentansprüche

1. PSD-95-Inhibitor, umfassend:
i. ein erstes Peptid (P₁), umfassend oder bestehend aus der Aminosäuresequenz X₂TX₃V (SEQ ID NO: 53), wobei
X₂ ausgewählt ist aus der Gruppe, bestehend aus E und S;
X₃ ausgewählt ist aus der Gruppe, bestehend aus L, T, V, R und D;
ii. ein zweites Peptid (P₂), umfassend oder bestehend aus der Aminosäuresequenz X₅TX₆V (SEQ ID NO: 55), wobei
X₅ ausgewählt ist aus der Gruppe, bestehend aus E und S;
X₆ ausgewählt ist aus der Gruppe, bestehend aus L, T, V, R und D; und
iii. ein zellpenetrierendes Peptid (CPP), ausgewählt aus der Gruppe, bestehend aus:
a) einem Poly-L-Arginin-Peptid (Poly-Arg) bestehend aus 3 bis 9 L-Argininresten;
b) yGrkkrrqrrr (SEQ ID NO: 9, D-TAT);
c) RQIKIWFQNRRMKWKK (SEQ ID NO: 14, L-Pen);
d) rqikiwfqnrrmkwkk (SEQ ID NO: 15, D-Pen);
e) RKKRRRESRKKRRRES (SEQ ID NO: 17, L-DPV3);
f) LLIILRRRIRKQAHAHSK (SEQ ID NO: 18, L-pVEC);
g) KLALKLALKALKAALKLA (SEQ ID NO: 16, L-MAP);
h) PLIYLRLLRGQF (SEQ ID NO: 19, L-TP2);
i) |(Dap)KAPETALD| (SEQ ID NO: 21, MiniAp4); und
j) |Ff(Nal2)RrRrQ|GABA-K (SEQ ID NO: 22, CPP12),
wobei das CPP über sein C-Terminus mit einem Linker verknüpft ist und P₁ und P₂ über ihre N-Termini mit dem Linker konjugiert sind und der PSD-95-Inhibitor die allgemeine Struktur der Formel (I) aufweist: 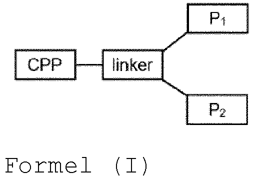
oder ein pharmazeutisch verträgliches Salz davon.

2. PSD-95-Inhibitor nach Anspruch 1, wobei P₁ die Sequenz X₁X₂TX₃V (SEQ ID NO: 54) umfasst oder daraus besteht, wobei
X₁ ausgewählt ist aus der Gruppe, bestehend aus K, V und I;
X₂ ausgewählt ist aus der Gruppe, bestehend aus E und S; und
X₃ ausgewählt ist aus der Gruppe, bestehend aus L, T, V, R und D;
und wobei P₂ die Sequenz X₄X₅TX₆V (SEQ ID NO: 56) umfasst oder daraus besteht, wobei
X₄ ausgewählt ist aus der Gruppe, bestehend aus K, V und I;
X₅ ausgewählt ist aus der Gruppe, bestehend aus E und S; und
X₆ ausgewählt ist aus der Gruppe, bestehend aus L, T, V, R und D.

3. PSD-95-Inhibitor nach einem der vorhergehenden Ansprüche, wobei P₁ und/oder P₂ aus 4 bis 10 Aminosäureresten bestehen, wie z. B. 5 Aminosäureresten.

4. PSD-95-Inhibitor nach einem der vorhergehenden Ansprüche, wobei X₃ und X₆ ausgewählt sind aus der Gruppe, bestehend aus L, T, V und R.

5. PSD-95-Inhibitor nach einem der vorhergehenden Ansprüche, wobei der Linker eine oder mehrere PEG-Einheiten umfasst, wobei mindestens ein Sauerstoffatom einer der PEG-Einheiten durch ein Stickstoffatom ersetzt ist, um NPEG zu ergeben, und wobei das CPP durch eine Amidbindung an das Stickstoffatom des Linkers gebunden ist und der PSD-95-Inhibitor die allgemeine Struktur der Formel (III) aufweist: 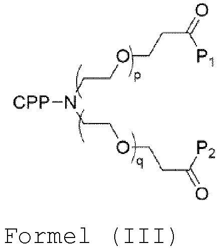 wobei
p eine Ganzzahl 0 bis 10 ist; und
q eine Ganzzahl 0 bis 10 ist.

6. PSD-95-Inhibitor nach einem der vorhergehenden Ansprüche, wobei P₁ und P₂ aus der Aminosäuresequenz KETLV (SEQ ID NO: 2), KETTV (SEQ ID NO: 3), KETVV (SEQ ID NO: 4), KETRV (SEQ ID NO: 5), ISTDV (SEQ ID NO: 6) oder VETVV (SEQ ID NO: 7) bestehen.

7. PSD-95-Inhibitor nach einem der vorhergehenden Ansprüche, wobei der PSD-95-Inhibitor die allgemeine Struktur der Formel (VI) aufweist: 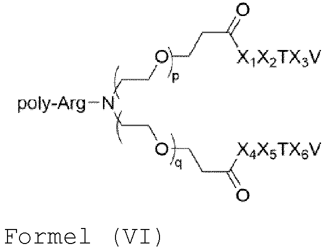 wobei
X₁ ausgewählt ist aus der Gruppe, bestehend aus K, V und I;
X₂ ausgewählt ist aus der Gruppe, bestehend aus E und S; und
X₃ ausgewählt ist aus der Gruppe, bestehend aus L, T, V, R und D.
X₄ ausgewählt ist aus der Gruppe, bestehend aus K, V und I;
X₅ ausgewählt ist aus der Gruppe, bestehend aus E und S;
X₆ ausgewählt ist aus der Gruppe, bestehend aus L, T, V, R und D.
p eine Ganzzahl 0 bis 10 ist;
q eine Ganzzahl 0 bis 10 ist; und
Poly-Arg ein Poly-L-Arginin-Peptid bestehend aus 3 bis 9 L-Argininresten ist.

8. PSD-95-Inhibitor nach einem der vorhergehenden Ansprüche, wobei das CPP RRRRRRRRR (SEQ ID NO: 12), RRRRRRRR (SEQ ID NO: 57), RRRRRRR (SEQ ID NO: 58), RRRRRR (SEQ ID NO: 59), RRRRR (SEQ ID NO: 60), RRRR (SEQ ID NO: 61) oder RRR ist.

9. PSD-95-Inhibitor nach einem der vorhergehenden Ansprüche, wobei der N-Terminus des CPP acetyliert ist oder wobei der N-Terminus des CPP an eine Chloralkan-Markierung (CA) konjugiert ist, die die folgende Struktur aufweist:

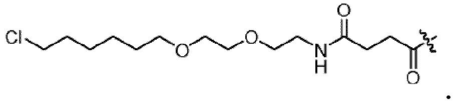

10. PSD-95-Inhibitor nach Anspruch 1, wobei der PSD-95-Inhibitor die allgemeine Struktur der Formel (XXVIII) aufweist: 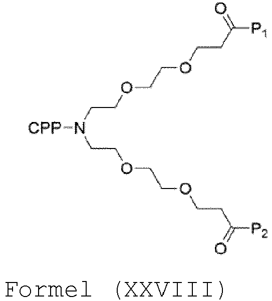
wobei P₁, P₂ und CPP Folgendes sind:
| | **P₁** | **P₂** | **CPP** | |
|---|---|---|---|---|
| i. | KETLV (SEQ ID NO: 2) | KETLV (SEQ ID NO: 2) | L-Arg₉ | RRRRRRRRR (SEQ ID NO: 12) |
| ii. | KETLV (SEQ ID NO: 2) | KETLV (SEQ ID NO: 2) | L-Arg₇ | RRRRRRR (SEQ ID NO: 58) |
| iii. | KETLV (SEQ ID NO: 2) | KETLV (SEQ ID NO: 2) | L-Arg₆ | RRRRRR (SEQ ID NO: 59) |
| iv. | KETLV (SEQ ID NO: 2) | KETLV (SEQ ID NO: 2) | L-Arg₅ | RRRRR (SEQ ID NO: 60) |
| V. | KETLV (SEQ ID NO: 2) | KETLV (SEQ ID NO: 2) | L-Arg₄ | RRRR (SEQ ID NO: 61) |
| vi. | KETLV (SEQ ID NO: 2) | KETLV (SEQ ID NO: 2) | L-Arg₃ | RRR |
| vii. | KETTV (SEQ ID NO: 3) | KETTV (SEQ ID NO: 3) | L-Arg₉ | RRRRRRRRR (SEQ ID NO: 12) |
| viii. | KETVV (SEQ ID NO: 4) | KETVV (SEQ ID NO: 4) | L-Arg₉ | RRRRRRRRR (SEQ ID NO: 12) |
| ix. | KETRV (SEQ ID NO: 5) | KETRV (SEQ ID NO: 5) | L-Arg₉ | RRRRRRRRR (SEQ ID NO: 12) |
| x. | ISTDV (SEQ ID NO: 6) | ISTDV (SEQ ID NO: 6) | L-Arg₉ | RRRRRRRRR (SEQ ID NO: 12) |
| xi. | VETVV (SEQ ID NO: 7) | VETVV (SEQ ID NO: 7) | L-Arg₉ | RRRRRRRRR (SEQ ID NO: 12) |
| xii. | IETDV (SEQ ID NO: 1) | IETDV (SEQ ID NO: 1) | L-Arg₉ | RRRRRRRRR (SEQ ID NO: 12) |
| xiii. | IETDV (SEQ ID NO: 1) | IETDV (SEQ ID NO: 1) | L-Arg₇ | RRRRRRR (SEQ ID NO: 58) |
| xiv. | IETDV (SEQ ID NO: 1) | IETDV (SEQ ID NO: 1) | L-Arg₅ | RRRRR (SEQ ID NO: 60) |
| xv. | IETDV (SEQ ID NO: 1) | IETDV (SEQ ID NO: 1) | L-Arg₃ | RRR |
und wobei der N-Terminus des CPP optional acetyliert ist
oder
wobei der PSD-95-Inhibitor die allgemeine Struktur der Formel (XXVIII) aufweist,
wobei P₁ und P₂ aus der Aminosäuresequenz IETDV (SEQ ID NO: 1) bestehen und das CPP ausgewählt ist aus der Gruppe, bestehend aus rqikiwfqnrrmkwkk (SEQ ID NO: 15, D-Pen); LLIILRRRIRKQAHAHSK (SEQ ID NO: 18, L-pVEC); PLIYLRLLRGQF (SEQ ID NO: 19, L-TP2); |(Dap)KAPETALD| (SEQ ID NO: 21, MiniAp4); und |Ff(Nal2)RrRrQ|GABA-K (SEQ ID NO: 22, CPP12).

11. PSD-95-Inhibitor nach Anspruch 1, wobei der PSD-95-Inhibitor ausgewählt ist aus der Gruppe, bestehend aus den Formeln (X) bis (XXIII):

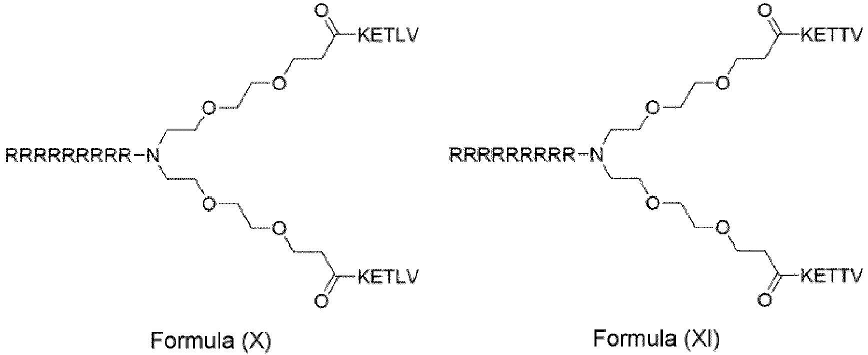

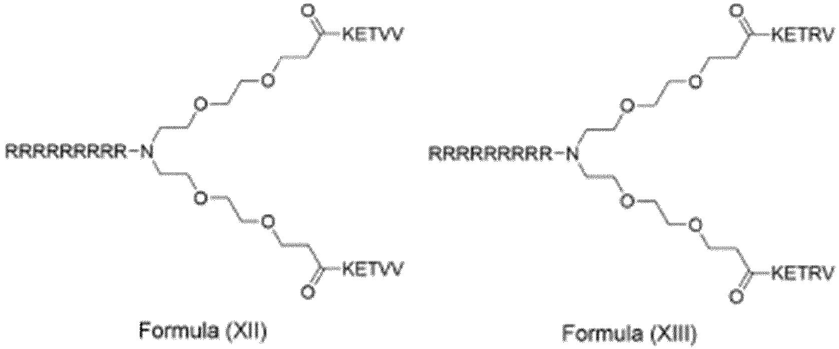

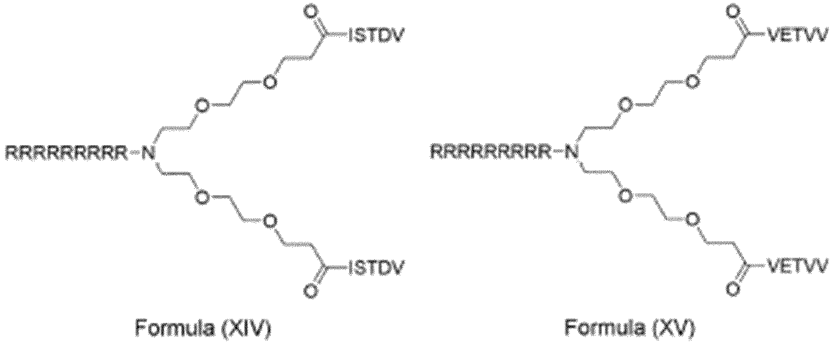

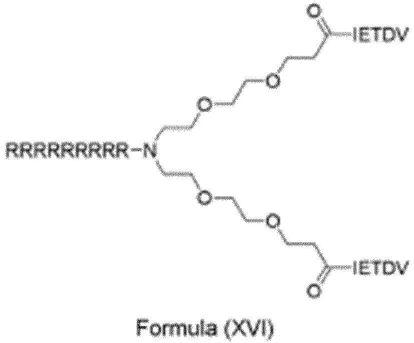

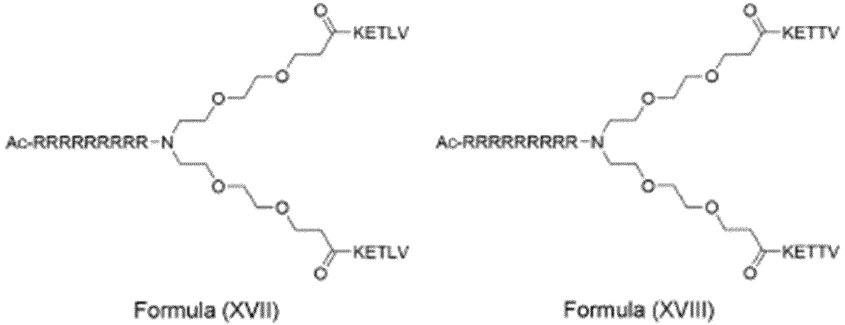

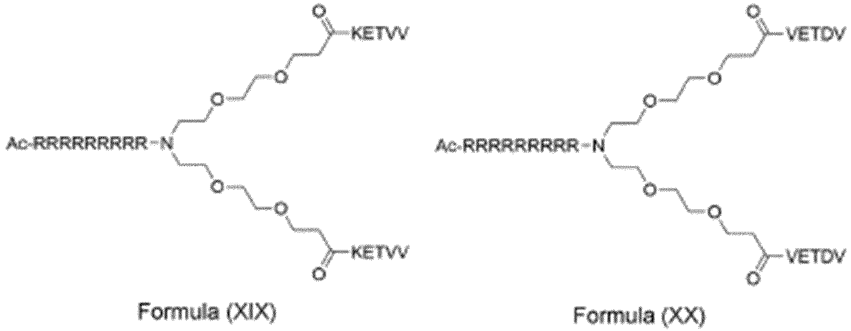

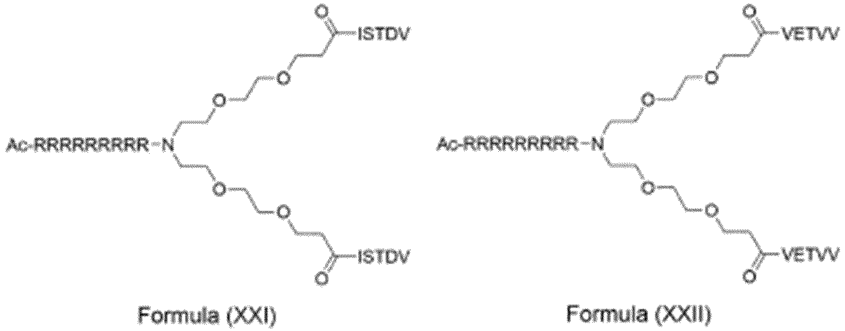

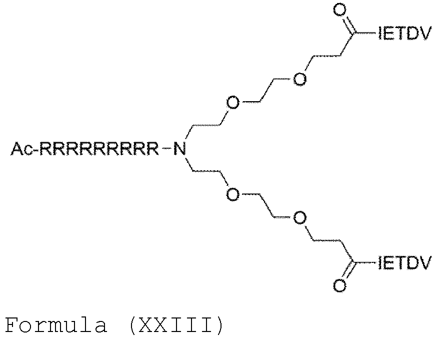

12. PSD-95-Inhibitor nach einem der Ansprüche 1 bis 3, 5, 9 oder 10, wobei P₁ und P₂ aus der Aminosäuresequenz IETDV (SEQ ID NO: 1) bestehen und das CPP ausgewählt ist aus der Gruppe, bestehend aus rqikiwfqnrrmkwkk (SEQ ID NO: 15, D-Pen); LLIILRRRIRKQAHAHSK (SEQ ID NO: 18, L-pVEC); PLIYLRLLRGQF (SEQ ID NO: 19, L-TP2); |(Dap)KAPETALD| (SEQ ID NO: 21, MiniAp4); und |Ff(Nal2)RrRrQ|GABA-K (SEQ ID NO: 22, CPP12).

13. PSD-95-Inhibitor nach einem der vorhergehenden Ansprüche zur Verwendung als Arzneimittel.

14. PSD-95-Inhibitor nach einem der Ansprüche 1 bis 12 zur Verwendung bei der Behandlung, Vorbeugung, Verringerung und/oder Verzögerung der Entwicklung einer exzitotoxisch-bedingten Erkrankung wie z. B. Schlaganfall, beispielsweise ausgewählt aus akutem ischämischem Schlaganfall und Subarachnoidalblutung.

15. Verfahren zum Herstellen des PSD-95-Inhibitors nach einem der Ansprüche 1 bis 12, umfassend die folgenden allgemeinen Schritte:
i.
a) Synthetisieren eines Peptids P₁/P₂;
b) Dimerisieren des Peptids von a) mit einem OPEGₙ- oder NPEGₙ-Linker, wobei "n" die Anzahl der PEG-Einheiten ist; und
c) Anbringen eines CPP-Tags an dem N-Terminus des NPEG-Linkers, beispielsweise unter Verwendung eines automatisierten Peptidsynthesizers;
oder
ii.
a) Bereitstellen eines Ns-NPEG-Disäure-Linkers;
b) Zubereiten eines Peptids P₁/P₂ unter Verwendung einer Fmocbasierten Festphasen-Peptidsynthese;
c) Dimerisieren des Fmoc-deprotectierten Peptids P₁/P₂ mit dem Ns-NPEG-Disäure-Linker, der ein Linker-Dimer-Konjugat bildet; und
d) Anbringen eines CPP an das N des NPEG-Linkers des Linker-Dimer-Konjugats, beispielsweise unter Verwendung eines automatisierten Peptidsynthesizers.

## Revendications

1. Inhibiteur de PSD-95 comprenant :
i. un premier peptide (P₁) comprenant ou constitué de la séquence d'acides aminés X₂TX₃V (SEQ ID NO: 53), dans lequel
X₂ est choisi dans un groupe composé de E et S ;
X₃ est choisi dans un groupe composé de L, T, V, R et D ;
ii. un second peptide (P₂) comprenant ou constitué de la séquence d'acides aminés X₅TX₆V (SEQ ID NO: 55), dans lequel
X₅ est choisi dans un groupe composé de E et S ;
X₆ est choisi dans un groupe composé de L, T, V, R et D ; et
iii. un peptide pénétrant dans les cellules (CPP) choisi dans un groupe composé de :
a) un peptide poly-L-arginine (poly-Arg) constitué de 3 à 9 résidus L-arginine ;
b) yGrkkrrqrrr (SEQ ID NO: 9, D-TAT) ;
c) RQIKIWFQNRRMKWKK (SEQ ID NO: 14, L-Pen) ;
d) rqikiwfqnrrmkwkk (SEQ ID NO: 15, D-Pen) ;
e) RKKRRRESRKKRRRES (SEQ ID NO: 17, L-DPV3) ;
f) LLIILRRRIRKQAHAHSK (SEQ ID NO: 18, L-pVEC) ;
g) KLALKLALKALKAALKLA (SEQ ID NO: 16, L-MAP) ;
h) PLIYLRLLRGQF (SEQ ID NO: 19, L-TP2) ;
i) |(Dap)KAPETALD| (SEQ ID NO: 21, MiniAp4) ; et
j) |Ff(Nal2)RrRrQ|GABA-K (SEQ ID NO: 22, CPP12),
dans lequel le CPP est lié à un lieur via son extrémité C-terminale, et P₁ et P₂ sont conjugués au lieur via leurs N-terminaux, et l'inhibiteur de PSD-95 a la structure générale de formule (I) : 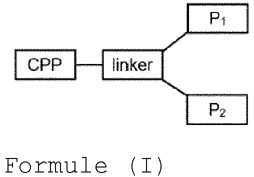
ou un sel pharmaceutiquement acceptable de celui-ci.

2. Inhibiteur de PSD-95 selon la revendication 1, dans lequel P₁ comprend ou est constitué de la séquence X₁X₂TX₃V (SEQ ID NO: 54), dans lequel
X₁ est choisi dans un groupe composé de K, V et I ;
X₂ est choisi dans un groupe composé de E et S ; et
X₃ est choisi dans un groupe composé de L, T, V, R et D ;
et dans lequel P₂ comprend ou est constitué de la séquence X₄X₅TX₆V (SEQ ID NO: 56), dans lequel
X₄ est choisi dans un groupe composé de K, V et I ;
X₅ est choisi dans un groupe composé de E et S ; et
X₆ est choisi dans un groupe composé de L, T, V, R et D.

3. Inhibiteur de PSD-95 selon l'une quelconque des revendications précédentes, dans lequel P₁ et/ou P₂ sont constitués de 4 à 10 résidus d'acides aminés, tels que 5 résidus d'acides aminés.

4. Inhibiteur de PSD-95 selon l'une quelconque des revendications précédentes, dans lequel X₃ et X₆ sont choisis dans un groupe composé de L, T, V et R.

5. Inhibiteur de PSD-95 selon l'une quelconque des revendications précédentes, dans lequel le lieur comprend une ou plusieurs unités PEG dans lequel au moins un atome d'oxygène d'une des unités PEG est remplacé par un atome d'azote pour donner du NPEG, et dans lequel le CPP est lié à l'atome d'azote du lieur par une liaison amide, et l'inhibiteur de PSD-95 a la structure générale de formule (III) : 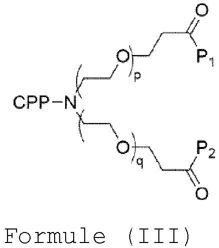 dans lequel
p est un entier compris entre 0 et 10 ; et
q est un entier compris entre 0 et 10.

6. Inhibiteur de PSD-95 selon l'une quelconque des revendications précédentes, dans lequel P₁ et P₂ sont constitués de la séquence d'acides aminés KETLV (SEQ ID NO: 2), KETTV (SEQ ID NO: 3), KETVV (SEQ ID NO: 4), KETRV (SEQ ID NO: 5), ISTDV (SEQ ID NO: 6), ou VETVV (SEQ ID NO: 7).

7. Inhibiteur de PSD-95 selon l'une quelconque des revendications précédentes, dans lequel l'inhibiteur de PSD-95 a la structure générale de formule (VI) : 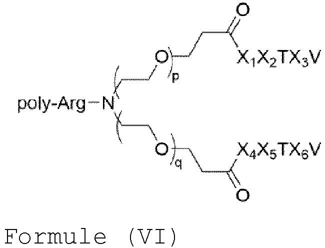 dans lequel
X₁ est choisi dans un groupe composé de K, V et I ;
X₂ est choisi dans un groupe composé de E et S ; et
X₃ est choisi dans un groupe composé de L, T, V, R et D.
X₄ est choisi dans un groupe composé de K, V et I ;
X₅ est choisi dans un groupe composé de E et S ;
X₆ est choisi dans un groupe composé de L, T, V, R et D.
p est un entier compris entre 0 et 10 ;
q est un entier compris entre 0 et 10 ; et
poly-Arg est un peptide poly-L-arginine constitué de 3 à 9 résidus L-arginine.

8. Inhibiteur de PSD-95 selon l'une quelconque des revendications précédentes, dans lequel le CPP est RRRRRRRRR (SEQ ID NO: 12), RRRRRRRR (SEQ ID NO: 57), RRRRRRR (SEQ ID NO: 58), RRRRRR (SEQ ID NO: 59), RRRRR (SEQ ID NO: 60), RRRR (SEQ ID NO: 61), ou RRR.

9. Inhibiteur de PSD-95 selon l'une quelconque des revendications précédentes, dans lequel l'extrémité N-terminale du CPP est acétylée ou dans lequel l'extrémité N-terminale du CPP est conjuguée à une étiquette de chloroalcane (CA), qui a la structure suivante :

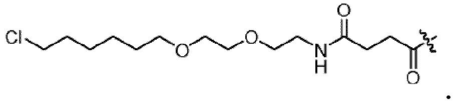

10. Inhibiteur de PSD-95 selon la revendication 1, dans lequel l'inhibiteur de PSD-95 a la structure générale de formule (XXVIII) : 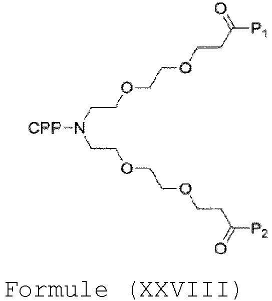
dans lequel P₁, P₂ et CPP sont :
| | **P₁** | **P₂** | **CPP** | |
|---|---|---|---|---|
| i. | KETLV (SEQ ID NO: 2) | KETLV (SEQ ID NO: 2) | L-Arg₉ | RRRRRRRRR (SEQ ID NO: 12) |
| ii. | KETLV (SEQ ID NO: 2) | KETLV (SEQ ID NO: 2) | L-Arg₇ | RRRRRRR (SEQ ID NO: 58) |
| iii. | KETLV (SEQ ID NO: 2) | KETLV (SEQ ID NO: 2) | L-Arg₆ | RRRRRR (SEQ ID NO: 59) |
| iv. | KETLV (SEQ ID NO: 2) | KETLV (SEQ ID NO: 2) | L-Arg₅ | RRRRR (SEQ ID NO: 60) |
| V. | KETLV (SEQ ID NO: 2) | KETLV (SEQ ID NO: 2) | L-Arg₄ | RRRR (SEQ ID NO: 61) |
| vi. | KETLV (SEQ ID NO: 2) | KETLV (SEQ ID NO: 2) | L-Arg₃ | RRR |
| vii. | KETTV (SEQ ID NO: 3) | KETTV (SEQ ID NO: 3) | L-Arg₉ | RRRRRRRRR (SEQ ID NO: 12) |
| viii. | KETVV (SEQ ID NO: 4) | KETVV (SEQ ID NO: 4) | L-Arg₉ | RRRRRRRRR (SEQ ID NO: 12) |
| ix. | KETRV (SEQ ID NO: 5) | KETRV (SEQ ID NO: 5) | L-Arg₉ | RRRRRRRRR (SEQ ID NO: 12) |
| x. | ISTDV (SEQ ID NO: 6) | ISTDV (SEQ ID NO: 6) | L-Arg₉ | RRRRRRRRR (SEQ ID NO: 12) |
| xi. | VETVV (SEQ ID NO: 7) | VETVV (SEQ ID NO: 7) | L-Arg₉ | RRRRRRRRR (SEQ ID NO: 12) |
| xii. | IETDV (SEQ ID NO: 1) | IETDV (SEQ ID NO: 1) | L-Arg₉ | RRRRRRRRR (SEQ ID NO: 12) |
| xiii. | IETDV (SEQ ID NO: 1) | IETDV (SEQ ID NO: 1) | L-Arg₇ | RRRRRRR (SEQ ID NO: 58) |
| xiv. | IETDV (SEQ ID NO: 1) | IETDV (SEQ ID NO: 1) | L-Arg₅ | RRRRR (SEQ ID NO: 60) |
| xv. | IETDV (SEQ ID NO: 1) | IETDV (SEQ ID NO: 1) | L-Arg₃ | RRR |
et dans lequel le N-terminal du CPP est éventuellement acétylé,
ou
dans lequel l'inhibiteur de PSD-95 a la structure générale de formule (XXVIII),
dans lequel P₁ et P₂ sont constitués de la séquence d'acides aminés IETDV (SEQ ID NO: 1) et le CPP est choisi dans un groupe composé de rqikiwfqnrrmkwkk (SEQ ID NO: 15, D-Pen) ; LLIILRRRIRKQAHAHSK (SEQ ID NO: 18, L-pVEC) ; PLIYLRLLRGQF (SEQ ID NO: 19, L-TP2) ; |(Dap)KAPETALD| (SEQ ID NO: 21, MiniAp4) ; et |Ff(Nal2)RrRrQ|GABA-K (SEQ ID NO: 22, CPP12).

11. Inhibiteur de PSD-95 selon la revendication 1, dans lequel l'inhibiteur de PSD-95 est choisi dans un groupe composé des formules (X) à (XXIII) :

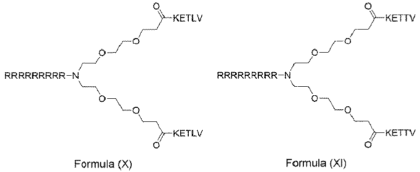

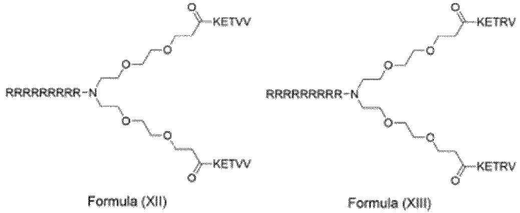

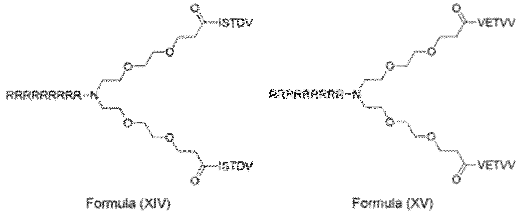

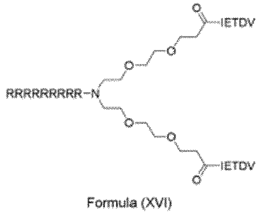

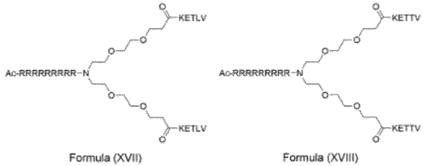

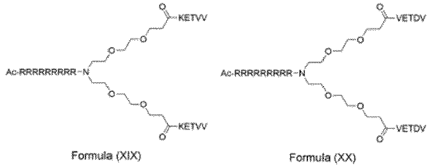

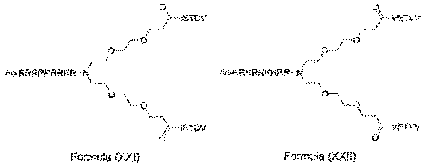

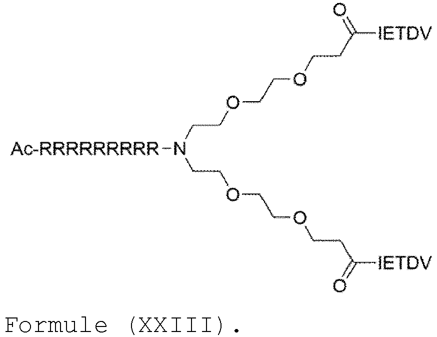

12. Inhibiteur de PSD-95 selon l'une quelconque des revendications 1 à 3, 5, 9 ou 10, dans lequel P₁ et P₂ sont constitués de la séquence d'acides aminés IETDV (SEQ ID NO: 1) et le CPP est choisi dans un groupe composé de rqikiwfqnrrmkwkk (SEQ ID NO: 15, D-Pen) ; LLIILRRRIRKQAHAHSK (SEQ ID NO: 18, L-pVEC) ; PLIYLRLLRGQF (SEQ ID NO: 19, L-TP2) ; |(Dap)KAPETALDI (SEQ ID NO: 21, MiniAp4) ; et |Ff(Nal2)RrRrQ|GABA-K (SEQ ID NO: 22, CPP12).

13. Inhibiteur de PSD-95 selon l'une quelconque des revendications précédentes pour une utilisation comme médicament.

14. Inhibiteur de PSD-95 selon l'une quelconque des revendications 1 à 12 pour une utilisation dans le traitement, la prévention, la réduction et/ou le retard du développement d'une maladie liée à l'excitotoxicité telle qu'un accident vasculaire cérébral, par exemple choisie parmi un accident vasculaire cérébral ischémique aigu et une hémorragie sous-arachnoïdienne.

15. Procédé de fabrication de l'inhibiteur de PSD-95 selon l'une quelconque des revendications 1 à 12 comprenant les étapes générales suivantes :
i.
a) synthèse d'un peptide P₁/P₂ ;
b) dimérisation du peptide de a) avec un lieur OPEGₙ ou NPEGₙ, dans lequel « n » est le nombre de fractions PEG ; et
c) Fixation d'une étiquette CPP au niveau du N du lieur NPEG, par exemple à l'aide d'un synthétiseur de peptides automatisé ; ou
ii.
a) fourniture d'un lieur diacide Ns-NPEG ;
b) préparation d'un peptide P₁/P₂ à l'aide d'une synthèse peptidique en phase solide à base de Fmoc,
c) dimérisation du peptide P₁/P₂-Fmoc déprotégé avec le lieur diacide Ns-NPEG formant un conjugué lieur-dimère ; et
d) fixation d'un CPP au N du lieur NPEG du conjugué lieur-dimère, par exemple à l'aide d'un synthétiseur de peptides automatisé.
